# EUROPEAN PATENT APPLICATION

(11) **EP 4 487 847 A1**
(43) Date of publication of application: **08.01.2025**
(21) Application number: 23763038.9
(22) Date of filing: 16.02.2023
(51) Int. Cl.: A61K 31/4184, A61K 31/4402, A61K 31/444, C07D 235/14, C07D 213/06

(54) **COMPOUNDS DERIVED FROM 4-AMINO-N-(4-AMINOPHENYL)BENZAMIDE AND ANALOGUES AND USE THEREOF**

(30) Priority: 04.03.2022 ES 202230181
(71) Applicant: Consejo Superior de Investigaciones Cientificas, 28006 Madrid (ES); Universidad Complutense De Madrid, 28040 Madrid (ES)
(72) Inventor: DARDONVILLE, Christophe, 28006 Madrid (ES); NUÉ MARTÍNEZ, Jorge Jonathan, 28006 Madrid (ES); CISNEROS CAÑAS, David, 28006 Madrid (ES); GAMARRO CONDE, Francisco, 18016 Granada (ES); MANZANO GONZÁLEZ, José Ignacio, 18016 Granada (ES); GÓMEZ BARRIO, Alicia, 28040 Madrid (ES); FONSECA BERZAL, Cristina Rosa, 28040 Madrid (ES); IBAÑEZ ESCRIBANO, Alexandra, 28040 Madrid (ES); ESCARIO GARCÍA-TREVIJANO, José Antonio, 28040 Madrid (ES); TORRADO DURÁN, Santiago, 28040 Madrid (ES); NOGAL RUIZ, Juan José, 28040 Madrid (ES)
(74) Representative: Pons
(86) International application number: PCT/ES2023/070084
(87) International publication number: WO 2023/166232

(57) **Abstract**

The present invention relates to compounds of formula (I), in addition to the use thereof for the treatment and/or prevention of diseases caused by protozoa, especially those belonging to the genus *Leishmania* or *Trypanosoma.* The present invention likewise relates to pharmaceutical compositions comprising said compounds.

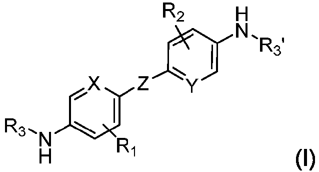

## Description

The present invention relates to 4-amino-*N*-(4-aminophenyl)benzamide derivatives and analogues, as well as the use thereof in the treatment of diseases, affecting both humans and animals, caused by protozoa such as trypanosomatids (leishmaniasis, trypanosomiasis). Therefore, the invention falls within the pharmaceutical and veterinary sector.

### BACKGROUND OF THE INVENTION

Human leishmaniasis is a disease that exists worldwide, where its prevalence has reached 12 million cases. It particularly appears in tropical and subtropical regions, although it is also found in Mediterranean countries. Furthermore, there is a strong incidence of infection by *Leishmania* in immunocompromised patients, especially among AIDS patients (Alvar, J. et al. Adv. Parasitol. 2006, 61, 223-74). Parasites of the genus *Leishmania* cause different forms of the disease which range from cutaneous leishmaniasis, which is milder, to visceral leishmaniasis, a much more serious form of the disease that is fatal if not treated properly. The species *Leishmania* that appears in the Mediterranean region and specifically in Spain is *L. infantum,* which causes cutaneous and visceral forms of the disease. Therapeutic options for treating leishmaniasis include pentavalent antimonial derivatives (sodium stibogluconate and meglumine antimoniate), the antibiotic amphotericin B and liposomal formulation thereof AmBisome^{®}, the antibiotic paromomycin, the alkylphosphocholine derivative miltefosine, 8-aminoquinoline sitamaquine and diamidine pentamidine (Mishra, J. et al. Curr. Med. Chem. 2007, 14, 1153-69). However, these drugs have several limitations such as their toxicity, their difficult administration, their high price and, the most worrying, the emergence of strains resistant to antimonials (Croft, S. L. et al. Clin Microbiol Rev 2006, 19, 111-26) and miltefosine (Srivastava, S. et al. Parasites & Vectors 2017,10, 49). Furthermore, the treatment of the most complex forms of the disease, as well as in cases of co-infection with HIV, is not very effective.

The pathogenic protozoan *Trypanosoma cruzi* is the etiologic agent of Chagas disease or American trypanosomiasis; this parasitosis is endemic to 21 Latin American countries, where it causes more than 7,000 deaths annually and keeps around 25 million people at risk of contracting the infection. In the area originally affected by the disease, it is transmitted mainly through the contaminated faeces of blood-sucking bugs of the Reduviidae family, although there are alternative routes of infection such as the oral route, transfusion of contaminated blood or the congenital route, among others. As a consequence of the international migrations that have occurred in recent decades, Chagas disease is an emerging pathology in countries such as the United States or Spain, currently affecting about 7 million people around the world.

After infection, the initial acute phase of Chagas disease is generally mild or asymptomatic and lasts 1-2 months. The infection persists throughout life, although 60-70% of infected individuals enter what is called the indeterminate phase of the disease, and never develop clinical manifestations. However, the remaining 30-40% develop the chronic phase of the disease 10-30 years after the initial infection, which is characterised by very serious cardiomyopathy and/or severe digestive problems (megacolon and megaesophagus).

There is no vaccine available for Chagas disease, and the only two drugs available, the nitroheterocycles nifurtimox and benznidazole, are effective in the acute phase, but they show limited efficacy in the chronic phase. Moreover, both compounds can produce severe side effects and, in addition, there are strains of the parasite that are naturally resistant to both drugs. There are currently no therapeutic alternatives that can replace nifurtimox and benznidazole.

Human African trypanosomiasis (HAT or sleeping sickness) is a parasitic infection, transmitted by the tsetse fly, caused by two subspecies of *Trypanosoma brucei: T. b. gambiense* and *T. b. rhodesiense.* Other species and subspecies of *Trypanosoma (T. congolense, T. brucei* subs. *brucei*) infect livestock, causing the disease called *nagana,* with great economic damage in sub-Saharan Africa.

Treatment options for sleeping sickness have been based for decades on relatively toxic drugs that require long administration protocols and hospitalisation of patients (H.P. de Koning, Trav. Med. Infect. Dis. 2020, 5(1), 14). In recent years, a new active drug taken orally has been registered, fexinidazole, for both stages of the gambiense HAT. Although the approval of fexinidazole constitutes an important breakthrough in the battle against the disease, the possible emergence of cross-resistance between these two drugs from the nitroimidazole family, fexinidazole and nifurtimox (both used for late-stage gambiense HAT) should not be overlooked. Furthermore, rhodesiense HAT remains the most neglected disease in the world with only suramin (early stage) and melarsoprol (an extremely toxic arsenic derivative) in propylene glycol to treat late-stage disease.

Moreover, the phenomenon of resistance to veterinary drugs (diminazene, ethidium bromide) used to treat animal trypanosomiasis in cattle is on the rise. Therefore, the discovery of new trypanocidal drugs for veterinary use is a high priority issue.

The *in vivo* activity of bisarylimidamides derived from 4,4'-(furan-2,5-diyl)dianiline on *Leishmania* and *Trypanosoma cruzi* has been described (Batista, D. D. G. J. Antimicrob. Agents Chemother. 2010, 54, 2940). Furthermore, bisarylimimamides derived from 4,4'-(furan-2,5-diyl)dianiline with activity on apicomplexa parasites *Besnoitia besnoiti, Neospora canine* and *Toxoplasma gondii* have been described (Kropf, C. Parasitology 2012, 139, 208). In addition, bisarylimidamides derived from benzidine, benzene-1,4-diamine, 4,4'-methylenedianiline, 4,4'-oxydianiline and 4,4'-(oxybis(methylene))dianiline with leishmanicidal activity (Banerjee, M. Eur. J. Med. Chem. 2012, 55, 449) and arylimidamides derived from 9*H-*carbazole-2,7-diamine, 4,4'-(ethyne-1,2-diyl)dianiline and 1,3-phenylenedimethanamine (Timm, B. L. Antimicrob. Agents Chemother. 2014, 58, 3720) or [1,1':3',1"-terphenyl]-diamine (Guedes-Da-Silva, F. H. Antimicrob. Agents Chemother. 2016, 60, 2425) on *T. cruzi* have been described. Moreover, mono-arylimidamides have been described with activity on *Leishmania* (Zhu, X. Bioorg. Med. Chem. Lett. 2016, 26, 2551).

WO2016033635 and WO2005051296 also relate to compounds for treating or preventing protozoan infection.

However, no bis-arylimidamide or bis-2-aminobenzimidazole compounds derived from N-phenylbenzamide, 1,3-diphenylurea, 1,2-diphenylethane, or *N*-(piridin-2-yl)picolinamide as leishmanicidal and tripanocidal agents.

Bis-2-aminoimidazoline derivatives with a skeleton of N-phenylbenzamide with activity on *T. brucei* have been described (Rodriguez et al J. Med. Chem. 2008, 51, 909-923). However, bis-2-aminoimidazoline derivatives with two substituents in the *N-*phenylbenzamide skeleton have not been described.

Some advantages of the compounds described in the present patent are their improved physico-chemical properties (that is, its greater lipophilicity and a reduced p*K*ₐ) that make them more bioavailable and, therefore, more potent against intracellular amastigote forms (that is, clinically relevant form in humans) of protozoan parasites, especially of the genera *Leishmania* and/or *Trypanosoma.* Furthermore, these compounds are more active and/or selective against these genera than the reference drugs miltefosine and benznidazole, respectively, such that they can be used against drug-resistant strains.

### DESCRIPTION OF THE INVENTION

The present invention discloses a family of compounds that have antiprotozoal activity in the micromolar range and that, therefore, are useful for the treatment of diseases caused by protozoa, preferably belonging to the genera *Leishmania* and *Trypanosoma.*

Therefore, a first aspect of the present invention relates to a compound of general formula (I), 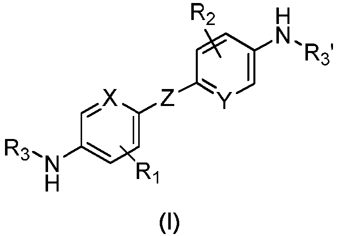
or any of the pharmaceutically acceptable isomers, solvates or salts thereof,
wherein:
   R₁ and R₂ are independently selected from H, O*ⁱ*Pr, and halogen;
   R₃ and R₃' are independently selected from 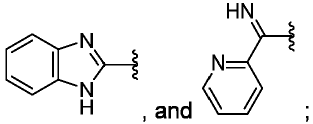
   Z is selected from 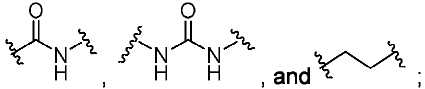
   X and Y are independently selected from CH and N,
   for use thereof as a medicament.

The term "solvate" is intended to indicate a pharmaceutically acceptable solvate form of a specified compound that maintains the biological efficacy of said compound. Examples of solvates include compounds of the invention together with water, isopropanol, ethanol, methanol, DMSO (dimethyl sulfoxide), ethyl acetate, acetic acid or ethanolamine. The term "hydrate" is used when said solvent is water. Solvation methods are generally known in the field.

The term "pharmaceutically acceptable salt" refers to salts prepared from pharmaceutically acceptable non-toxic bases including inorganic bases and organic bases. Salts derived from inorganic bases include salts of aluminium, ammonium, calcium, copper, ferric salts, ferrous salts, lithium, magnesium, manganic salts, manganous salts, potassium, sodium, zinc and the like. Salts derived from pharmaceutically acceptable non-toxic organic bases include salts of primary, secondary and tertiary amines, substituted amines including natural substituted amines, cyclic amines, and basic ion exchange resins, such as arginine, betaine, caffeine, choline, *N,N'*-dibenzylethylenediamine, diethylamine, 2-diethylaminoethanol, 2-dimethylaminoethanol, ethanolamine, ethylenediamine, *N*-ethyl-morpholine, *N-*ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. When the compound of the present invention is basic, salts can be prepared from pharmaceutically acceptable non-toxic acids, including inorganic and organic acids. Such acids include acetic acid, benzenesulphonic acid, benzoic acid, camphorsulphonic acid, citric acid, ethanesulphonic acid, fumaric acid, gluconic acid, glutamic acid, hydrobromic acid, hydrochloric acid, isethionic acid, lactic acid, maleic acid, malic acid, mandelic acid, methanesulphonic acid, mucic acid, nitric acid, pamoic acid, pantothenic acid, phosphorus acid, succinic acid, sulphuric acid, tartaric acid, p-toluenesulphonic acid and the like.

Preferred examples of pharmaceutically acceptable salts include salts of ammonium, calcium, magnesium, potassium and sodium, and those formed from maleic acid, fumaric acid, benzoic acid, ascorbic acid, pamoic acid, succinic acid, hydrochloric acid, sulphuric acid, bismethylenesalicylic acid, methanesulphonic acid, ethanedisulphonic acid, propionic acid, tartaric acid, salicylic acid, citric acid, gluconic acid, aspartic acid, stearic acid, palmitic acid, itaconic acid, glycolic acid, p-aminobenzoic acid, glutamic acid, benzenesulphonic acid, cyclohexylsulfamic acid, phosphoric acid and nitric acid.

The term isomer mainly refers to structural isomers, such as tautomers. Specifically, the term tautomer refers to one of two or more structural isomers of a compound that exist in equilibrium and are easily converted from one isomeric form to another. Common tautomeric pairs are, for example, amine-imine.

The term "halide" refers to F, Cl, Br or I.

Hereinafter, the compounds of formula (I), the pharmaceutically acceptable isomers, solvates or salts thereof will be referred to as compounds of the invention.

In a preferred embodiment of the compounds of the invention for use thereof, R₁ and R₂ are the same and, more preferably, R₁ and R₂ are H.

In another preferred embodiment, R₁ and R₂ are Cl.

In another preferred embodiment, R₁ and R₂ are O'Pr.

In another preferred embodiment, R₁ and R₂ are F.

In a preferred embodiment, X and Y are CH.

In another preferred embodiment, X is CH and Y is N.

In another preferred embodiment, X and Y are both N.

In a preferred embodiment, Z is 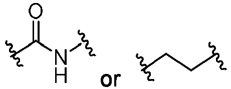 and, more preferably, Z is

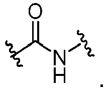

In another embodiment, Z is

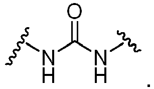

In a preferred embodiment, R₃ and R₃' are the same.

In a preferred embodiment, R₃ and R₃' are the same and are:

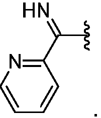

In another preferred embodiment, Z is 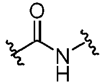 and R₃ and R₃' are:

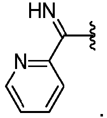

In another preferred embodiment, Z is 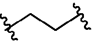 and R₃ and R₃' are:

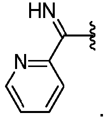

In another preferred embodiment, R₃ and R₃' are the same and are:

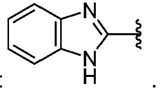

In another preferred embodiment, Z is 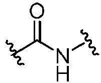 and R₃ and R₃' are

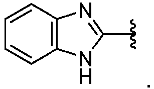

In another preferred embodiment, the compounds of the invention for use thereof are selected from the list comprising:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(2-chloro-4-(picolinimidamido)phenyl)-4-(picolinimidamido)benzamide **(3e)**
- 2-chloro-*N*-(3-chloro-4-(picolinimidamido)phenyl)-4-(picolinimidamido)benzamide **(3f)**
- 2-isopropoxy-*N*-(2-isopropoxy-4-(picolinimidamido)phenyl)-4-(picolinimidamido)benzamide **(3g)**
- 4-(picolinimidamido)*-N*-(5-(picolinimidamido)pyridin-2-yl)benzamide **(3h)**
- 3-fluoro-*N*-(2-fluoro-4-(picolinimidamido)phenyl)-4-(picolinimidamido)benzamide di-trifluoroacetate **(3i)**
- 3-fluoro*-N*-(3-fluoro-4-(picolinimidamido)phenyl)-4-(picolinimidamido)benzamide di-trifluoroacetate **(3j)**
- 2-fluoro-*N-*(2-fluoro-4-(picolinimidamido)phenyl)-4-(picolinimidamido)benzamide di-trifluoroacetate **(3k)**
- 2-fluoro-*N*-(3-fluoro-4-(picolinimidamido)phenyl)-4-(picolinimidamido)benzamide di-trifluoroacetate **(3l)**
- *N,N*"-(ethane-1,2-diylbis(4,1-phenylene))dipicolinimidamide **(17)**
- *N,N*"-((carbonylbis(azanediyl))bis(4,1-phenylene))dipicolinimidamide **(18)**
- 4-((1,3-dihydro-2*H*-benzo[*d*]imidazol-2-ylidene)amino)-*N*-(4-((1,3-dihydro-2*H-*benzo[dJimidazol-2-ylidene)amino)phenyl)benzamide **(1a)**
- 5-((1,3-dihydro-2*H*-benzo[*d*]imidazol-2-ylidene)amino)-*N*-(5-((1,3-dihydro-2*H-*benzo[*d*]imidazol-2-ylidene)amino)pyridin-2-il)picolinamide **(1b)**
- 3-chloro-*N*-(2-chloro-4-((1,3-dihydro-2*H*-benzo[*d*]imidazol-2-ylidene)amino)phenyl)-4-((1,3-dihydro-2*H*-benzo[d]imidazol-2-ylidene)amino)benzamide **(1c)**
- 3-chloro-*N*-(3-chloro-4-((1,3-dihydro-2*H*-benzo[*d*]imidazol-2-ylidene)amino)phenyl)-4-((1,3-dihydro-2*H*-benzo[d]imidazol-2-ylidene)amino)benzamide **(1d)**
- 2-chloro-*N*-(2-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1e)**
- 2-chloro-*N*-(3-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1f)**
- 4,4'-(ethane-1,2-diyl)bis(*N*-(1*H*-benzo[*d*]imidazol-2(3*H*)-ylidene)aniline **(13)**
- 1,3-bis(4-((1,3-dihydro-2*H*-benzo[*d*]imidazol-2-ylidene)amino)phenyl)urea **(14)**.

Another aspect of the present invention relates to a compound of formula (I) or any of the pharmaceutically acceptable isomers, solvates or salts thereof, as defined in the first aspect of the invention, for use thereof in the treatment and/or prevention of diseases caused by protozoan parasites. These diseases occur in an animal, preferably a mammal, including humans.

The term "treatment or prevention" as used herein, unless indicated otherwise, means reversing, alleviating, inhibiting the progress of, or preventing the disorder or condition to which it applies in such terms, one or more symptoms of such disorder or condition.

In a preferred embodiment, the compounds for use are administered to an animal, more preferably a mammal. In another preferred embodiment, the mammal is a human.

In a preferred embodiment, the protozoan parasites are of the genus *Leishmania* or *Trypanosoma.* Even more preferably, the parasites are of the species *L. aethiopica, L. amazonensis, L. arabica, L. archibaldi, L. aristedesi, L. braziliensis, L. chagasi (syn. L. infantum), L. colombiensis, L. deanei, L. donovani, L. enriettii, L. equatorensis, L. forattinii, L. gamhami, L. gerbili, L. guyanensis, L. herreri, L. hertigi, L. infantum, L. killicki, L. lainsoni, L. major, L. mexicana, L. naiffi, L. panamensis, L. peruviana, L. pifanoi, L. shawi, L. tarentolae, L. tropica, L. turanica, L. venezuelensis, T. brucei, T. cruzi, T. congolense, T. equinum, T. equiperdum, T. evansi, T. vivax.* Even more preferably, the parasites are of the species *T. brucei, T. cruzi* and *L. donovani.*

In another preferred embodiment, the disease caused by protozoan parasites is selected from leishmaniasis, human African trypanosomiasis (sleeping sickness), animal trypanosomiasis, or American trypanosomiasis (Chagas disease).

In a preferred embodiment, the invention relates to a compound selected from:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(3-chloro-4-(picolinimidamido)phenyl)-4-(picolinimidamido)benzamide **(3f)**
- 2-isopropoxy-*N*-(2-isopropoxy-4-(picolinimidamido)phenyl)-4-(picolinimidamido)benzamide **(3g)**
- 4-(picolinimidamido)-*N*-(5-(picolinimidamido)pyridin-2-yl)benzamide **(3h)**
- *N*,*N*"-(ethane-1,2-diylbis(4,1-phenylene))dipicolinimidamide **(17),**
more preferably:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- *N,N*"-(ethane-1,2-diylbis(4,1-phenylene))dipicolinimidamide **(17),**
for use thereof in the treatment and/or prevention of diseases caused by protozoan parasites of the genus *Leishmania,* more preferably of the species *L. donovani* and, even more preferably, of the species *L. donovani* in the promastigote and/or intracellular amastigote forms.

Another preferred embodiment of the invention relates to a compound selected from:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N-*(3-chloro-4-(picolinimidamido)phenyl)-4-(picolinimidamido)benzamide **(3d)** for use thereof in the treatment and/or prevention of diseases caused by protozoan parasites of the genus *Trypanosoma,* more preferably, of the species *T. brucei,* even more preferably, of the sensitive strain *T. brucei* s427 and/or the drug-resistant strain *T. brucei* B48.

In a preferred embodiment, the invention relates to a compound selected from:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(2-chloro-4-(picolinimidamido)phenyl)-4-(picolinimidamido)benzamide **(3e)**
- 2-chloro-*N*-(3-chloro-4-(picolinimidamido)phenyl)-4-(picolinimidamido)benzamide **(3f)**
- 2-isopropoxy-*N*-(2-isopropoxy-4-(picolinimidamido)phenyl)-4-(picolinimidamido)benzamide **(3g)**
- *N*,*N*"-(ethane-1,2-diylbis(4,1-phenylene))dipicolinimidamide **(17)**
- 3-fluoro-*N*-(2-fluoro-4-(picolinimidamido)phenyl)-4-(picolinimidamido)benzamide di-trifluoroacetate **(3i)**
- 3-fluoro*-N*-(3-fluoro-4-(picolinimidamido)phenyl)-4-(picolinimidamido)benzamide di-trifluoroacetate **(3j)**
- 2-fluoro-*N-*(2-fluoro-4-(picolinimidamido)phenyl)-4-(picolinimidamido)benzamide di-trifluoroacetate **(3k)**
- 2-fluoro-*N*-(3-fluoro-4-(picolinimidamido)phenyl)-4-(picolinimidamido)benzamide di-trifluoroacetate **(3l),**
more preferably:
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(3-chloro-4-(picolinimidamido)phenyl)-4-(picolinimidamido)benzamide **(3f)** for use thereof in the treatment and/or prevention of diseases caused by protozoan parasites of the genus *Trypanosoma,* more preferably, of the species *T. cruzi.*

Another aspect of the present invention relates to a compound of general formula (I) or any of the pharmaceutically acceptable isomers, solvates or salts thereof, as described in the first aspect of the invention, with the condition that the compound is not: 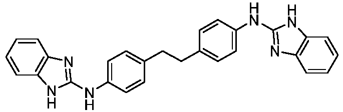 nor any of the tautomers thereof.

The preferred embodiments of the compounds of the invention referred to in this last aspect of the invention are those already mentioned in the first aspect of the invention that referred to said compounds for use thereof.

Unless otherwise indicated, the compounds of the invention include compounds that differ only in the presence of one or more isotopically enriched atoms. For example, compounds that have said structure, with the exception of the substitution of a hydrogen for deuterium or tritium, or the substitution of a carbon by a carbon enriched in ¹³C or ¹⁴C or a nitrogen enriched in ¹⁵N, are within the scope of this invention.

The present invention further relates to a pharmaceutical composition comprising a therapeutically effective amount of at least one compound of formula (I) as previously defined, any of the pharmaceutically acceptable isomers, solvates or salts thereof, with a pharmaceutically acceptable excipient, adjuvant and/or carrier.

The expression "excipients, adjuvants and/or carriers" refers to molecular entities or substances with which the active ingredient is administered. Such pharmaceutical excipients, adjuvants or carriers can be sterile liquids, such as water and oil, including those of petroleum or animal, plant or synthetic origin. such as peanut oil, soy oil, mineral oil, sesame oil and the like, excipients, disintegrants, wetting or thinning agents.

The expression "therapeutically effective amount" refers to the amount of the agent or compound capable of developing the therapeutic action determined by the pharmacological properties thereof, calculated to produce the desired effect and, in general, will be determined, among other causes, by the very characteristics of the compounds, as well as the age and condition of the patient, the severity of the illness or disorder, and the route and frequency of administration.

Said therapeutic composition can be prepared in solid or suspension form, in a pharmaceutically acceptable diluent. The therapeutic composition provided by this invention can be administered via any suitable route of administration, for which said composition will be formulated in the pharmaceutical form suitable for the chosen route of administration.

The compounds described in the present invention for use thereof, as well as the pharmaceutical compositions containing them can be used together with other additional drugs in order to provide a combination therapy. Said additional drugs can make up part of the same pharmaceutical composition or, alternatively, they can be provided in the form of a separate composition for the administration thereof which is simultaneous or not to that of the pharmaceutical composition comprising a compound of the invention.

Therefore, in a preferred embodiment, the composition of the invention further comprises at least one other active ingredient. Preferably, this active ingredient is an antiparasitic agent, more preferably, it is an antiprotozoal agent.

The present invention relates to the use of a compound of formula (I), or any of the isomers thereof or the pharmaceutically acceptable salts thereof, for the preparation of a medicament, preferably for use thereof in the treatment and/or prevention of diseases caused by protozoan parasites.

The present invention further relates to a method for the treatment and/or prevention of diseases caused by protozoan parasites that comprises administering a therapeutically effective amount of a compound of formula (I) or any of the pharmaceutically acceptable isomers, solvates or salts thereof to a subject (human or animal).

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples are provided by way of illustration, and are not meant to limit the present invention.

### EXAMPLES

Next, the invention will be illustrated by means of assays carried out by the inventors, which demonstrate the specificity and effectiveness of the compounds described in the present invention.

### Example 1. Synthesis of compounds of formula (I) according to the present invention

Scheme 1: Synthesis of compounds of the invention of formula (I) (compounds within the formulae **1** and **3** indicated in scheme 1) where Z is -CONH- in said formula.

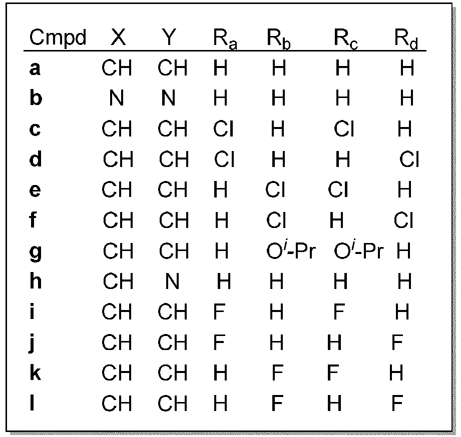

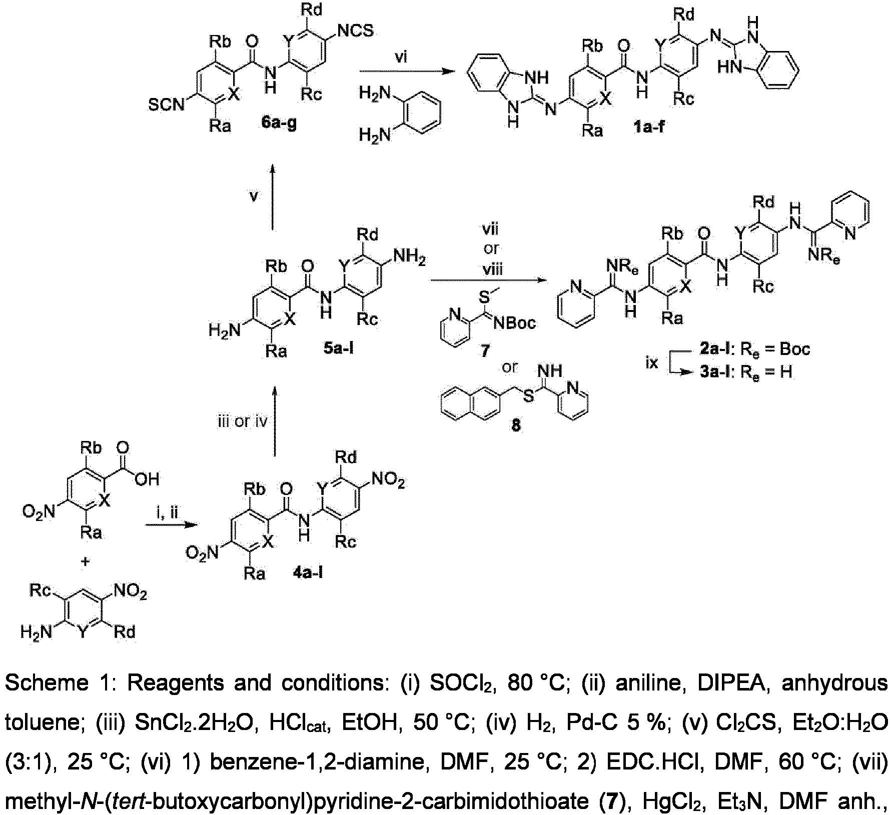

50 °C; (viii) naphthalen-2-ylmethyl pyridine-2-carbimidothioate hydrobromide (**8**), EtOH/CH₃CN (3:1), 0 °C→25 °C; (ix) CF₃CO₂H, CH₂Cl₂, 0 °C.

Scheme 2: Synthesis of compounds of the invention **13** and **17** of formula (I) where Z is -CH₂CH₂- in said formula and **14** and **18** where Z is -NHCONH-.

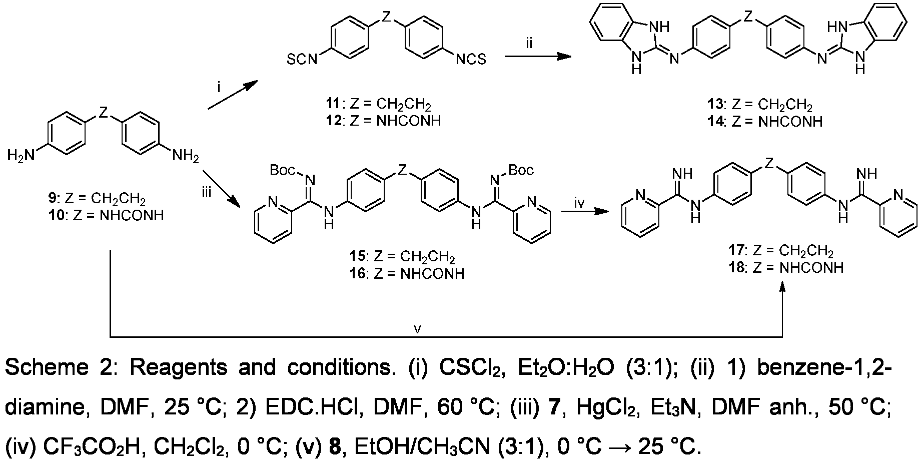

### 1.1. General method for the synthesis of N-phenylbenzamides 4a-l

The reactions were carried out in high pressure-resistant screw cap flasks. A solution of carboxylic acid (all carboxylic acids used are commercially available) (1.5 eq.) in SOCl₂ (25 mL) was stirred at 80 °C for the time indicated in each case. Once at room temperature, the flask is carefully opened, due to the internal pressure of SO₂ and HCl. The solution is concentrated under vacuum to remove excess SOCl₂, CH₂Cl₂ is added to the flask to remove traces of SOCl₂ and the solvent is removed. The acid chloride that is obtained as an oil is slowly added with the help of a syringe to a solution under stirring of nitroaniline (1.0 eq.) and DIPEA (2.0 eq.) in anhydrous toluene (20 mL) under an Argon atmosphere. The reaction mixture is stirred overnight at room temperature. Then the solvent is removed under vacuum and MeOH is added to the crude, to facilitate the precipitation of a solid. The precipitate is plate filtered and washed successively (3x) with 0.1 M aqueous HCl (10 mL) and MeOH (20 mL) to obtain the product as a powdered solid.

**5-nitro-N-(5-nitropyridin-2-yl)picolinamide (4b).** A catalytic amount of DMF is added drop by drop (10 drops), under an argon atmosphere, to a suspension of 5-aminopicolinic acid (1.9 g; 11.2 mmol) in anhydrous dichloromethane (10 mL) cooled with an ice bath. Next, oxalyl chloride (1.4 g; 1 mL; 11.2 mmol) is added dropwise and the reaction mixture is stirred at room temperature overnight. Next, Et₃N is added to the reaction mixture cooled with an ice bath, followed by the slow addition of a solution of 5-nitropyridine-2-amine (1.1 g; 8 mmol) in anhydrous toluene (15 mL). The reaction mixture was stirred for 12 hours at room temperature. Next, it is cooled with an ice bath and cold methanol is added to precipitate the product as a greyish solid (1.9 g; 81%). ¹H NMR (300 MHz, DMSO-*d*₆) δ 11.02 (s, 1H), 9.51 (d, *J* = 2.4 Hz, 1H), 9.25 (d, *J* = 2.7 Hz, 1H), 8.86 (dd, *J* = 8.6, 2.4 Hz, 1H), 8.74 (dd, *J* = 9.2, 2.7 Hz, 1H), 8.46 (d, *J =* 9.2 Hz, 1H), 8.43 (d, *J =* 8.6 Hz, 1H). ¹³C NMR (75 MHz, DMSO-*d*₆) δ 161.4, 154.5, 152.3, 146.3, 145.0, 144.2, 140.8, 134.8, 134.0, 123.7, 113.1. HPLC (UV) > 95 %. LRMS (ESI⁻): *m*/*z* 288.3 [M-H].

**3-chloro-N-(2-chloro-4-nitrophenyl)-4-nitrobenzamide (4c).** A mixture of 3-chloro-4-nitrobenzoic acid (850 mg; 1.5 mmol) and SOCl₂ (5 mL) reacted following the general method. After 12 h of reaction, the acid chloride was obtained in the form of yellowish oil. The acid chloride was added to a suspension of 2-chloro-4-nitroaniline (485 mg; 2.8 mmol) and DIPEA (728 mg; 0.98 mL; 5.6 mmol) in anhydrous toluene (20 mL). The reaction mixture was stirred at room temperature under an argon atmosphere for 16 h, obtaining the compound **4c** as a brown solid (760 mg; 76 %). ¹H NMR (300 MHz, DMSO-*d*₆) δ 10.73 (s, 1H), 8.44 (d, *J =* 2.6 Hz, 1H), 8.32 (d, *J =* 1.8 Hz, 1H), 8.29 (dd, *J* = 8.9, 2.6 Hz, 1H), 8.27 (d, *J* = 8.5 Hz, 1H), 8.13 (dd, *J* = 8.5, 1.8 Hz, 1H), 8.02 (d, *J =* 8.9 Hz, 1H). ¹³C NMR (101 MHz, DMSO) δ 163.2, 149.5, 145.1, 140.7, 138.1, 131.0, 128.7, 128.3, 127.5, 125.9, 125.2, 125.1, 123.0. HPLC (UV) > 95 %. LRMS (ESI⁻): *m*/*z* 354.3 [M-H].

**3-chloro-N-(3-chloro-4-nitrophenyl)-4-nitrobenzamide (4d)**. A mixture of 3-chloro-4-nitrobenzoic acid (3 g; 15 mmol) and SOCl₂ (50 mL) reacted following the general method. After 12 h of reaction, the acid chloride was obtained as a dark yellow oil. The acid chloride was added to a suspension of 3-chloro-4-nitroaniline (1.7 g; 10 mmol) and DIPEA (2.6 g; 3.5 mL; 20 mmol) in anhydrous toluene (20 mL). The reaction mixture was stirred at room temperature under an argon atmosphere for 16 h, obtaining the compound **4d** as a brown solid (2.7 g; 77%). ¹H NMR (300 MHz, DMSO-*d*₆) δ 11.09 (s, 1H), 8.32 (d, *J* = 1.8 Hz, 1H), 8.27 (d, *J* = 8.4 Hz, 1H), 8.20 (d, *J* = 2.4 Hz, 1H) 8.20 (d, *J* = 8.9 Hz, 1H), 8.12 (dd, *J* = 8.4, 1.8 Hz, 1H), 7.93 (dd, *J* = 8.9, 2.4 Hz, 1H). ¹³C NMR (75 MHz, DMSO-*d*₆) δ 163.5, 149.4, 143.5, 142.2, 138.5, 130.9, 128.3, 127.5, 126.6, 125.9, 125.1, 121.7, 119.0. HPLC (UV) > 95 %. LRMS (ESI⁻): *m*/*z* 354.3 [M-H].

**2-chloro-*N*-(2-chloro-4-nitrophenyl)-4-nitrobenzamide (4e).** A mixture of 2-chloro-4-nitrobenzoic acid (1.5 g; 7.5 mmol) and SOCl₂ (30 mL) reacted following the general method. After 12 h of reaction, the acid chloride was obtained in the form of yellowish oil. The acid chloride was added to a suspension of 2-chloro-4-nitroaniline (863 mg; 5 mmol) and DIPEA (1.5 g; 11.25 mmol) in anhydrous toluene (10 mL). The reaction mixture was stirred at room temperature under an argon atmosphere for 24 h, obtaining the compound **4e** as a brown solid (1.5 g; 83%). ¹H NMR (300 MHz, DMSO-ds) δ 10.89 (s, 1H), 8.43 (d, *J* = 2.2 Hz, 1H), 8.41 (d, *J* = 2.6 Hz, 1H), 8.32 (dd, *J* = 8.4, 2.2 Hz, 1H), 8.30 (dd, *J* = 9.0, 2.6 Hz, 1H), 8.22 (d, *J* = 9.0 Hz, 1H), 7.96 (d, *J* = 8.4 Hz, 1H). ¹³C NMR (75 MHz, DMSO-*d*₆) δ 164.3, 148.6, 144.6, 141.4, 140.2, 131.3, 130.3, 126.8, 125.9, 125.1, 124.6, 123.1, 122.5. HPLC (UV) > 95 %. LRMS (ESI⁻): *m*/*z* 354.2 [M-H].

**2-chloro-N-(3-chloro-4-nitrophenyl)-4-nitrobenzamide (4f).** A mixture of 2-chloro-4-nitrobenzoic acid (1.7 g; 8.4 mmol) and SOCl₂ (20 mL) reacted following the general method. After 12 h of reaction, the acid chloride was obtained in the form of yellowish oil. The acid chloride was added to a suspension of 3-chloro-4-nitroaniline (970 mg; 5.6 mmol) and DIPEA (1.5 g; 2.0 mL; 11.3 mmol) in anhydrous toluene (10 mL). The reaction mixture was stirred at room temperature under an argon atmosphere for 12 h, obtaining the compound **4f** as a yellowish solid (1.3 g; 65 %). ¹H NMR (300 MHz, DMSO-*d*₆) δ 11.41 (s, 1H), 8.46 (d, *J* = 2.2 Hz, 1H), 8.34 (dd, *J* = 8.4, 2.2 Hz, 1H), 8.20 (d, *J* = 9.0 Hz, 1H), 8.13 (d, *J =* 2.2 Hz, 1H), 7.98 (d, *J=* 8.4 Hz, 1H), 7.79 (dd, *J* = 9.0, 2.2 Hz, 1H). ¹³C NMR (75 MHz, DMSO-*d*₆) δ 164.3, 148.8, 143.1, 142.4, 141.2, 131.2, 130.3, 127.7, 126.9, 124.8, 122.7, 121.1, 118.5. HPLC (UV) > 95 %. LRMS (ESI⁻): *m*/*z* 354.3 [M-H].

**2-isopropoxy-*N*-(3-isopropoxy-4-nitrophenyl)-4-nitrobenzamide (4g).** A mixture of 2-isopropoxy-4-nitrobenzoic acid (0.5 g, 2.2 mmol) and SOCl₂ reacted following the general method. After 12 h of reaction, the acid chloride was obtained as a yellowish oil. The acid chloride was added to a suspension of 2-isopropoxy-4-nitroaniline (291 mg; 1.5 mmol) and DIPEA (383 mg; 0.5 mL; 3.0 mmol) in anhydrous toluene (10 mL). The reaction mixture was stirred at room temperature under an argon atmosphere for 48 h, obtaining the compound **4g** as a yellowish solid (410 mg; 69 %) by recrystallisation in MeOH. ¹H NMR (300 MHz, DMSO-*d₆*) δ 10.07 (s, 1H), 8.65 (d, *J* = 8.9 Hz, 1H), 8.15 (d, *J* = 8.6 Hz, 1H), 8.00 (d, *J* = 2.2 Hz, 1H), 7.95 (d, *J* = 2.2 Hz, 1H), 7.93 - 7.89 (m, 2H), 5.06 (d, *J* = 6.0 Hz, 1H), 4.95 (d, *J* = 6.0 Hz, 1H), 1.44 (d, *J* = 6.0 Hz, 6H), 1.39 (d, *J =* 6.0 Hz, 6H). ¹³C NMR (75 MHz, DMSO-d6) δ 162.8, 156.0, 150.8, 147.0, 143.6, 134.5, 133.1, 128.9, 120.3, 117.2, 116.1, 110.4, 108.5, 74.3, 73.0, 22.0.

**3-fluoro-N-(2-fluoro-4-nitrophenyl)-4-nitrobenzamide (4i)**. A mixture of 3-fluoro-4-nitrobenzoic acid (1.13 g, 6.1 mmol) and SOCl₂ (5 mL) reacted following the general method. After 17 h of reaction, the acid chloride was obtained in the form of a yellowish solid. The acid chloride 610 mg, 3 mmol) dissolved in anhydrous THF (2 mL) was added to a suspension of 2-fluoro-4-nitroaniline (312 mg, 2 mmol) and DIPEA (0.7 mL, 4 mmol) in anhydrous THF (5 mL). The reaction mixture was stirred at room temperature under an argon atmosphere for 3 days, obtaining the compound **4i** as a whitish solid (620 mg, 96 %). M.p. 204.3 °C. ¹H NMR (300 MHz, DMSO-*d*₆) δ 11.06 (s, 1H), 8.43 - 8.12 (m, 6H), 8.00 (dd, *J =* 7.0, 1.3 Hz, 1H). ¹³C NMR (75 MHz, DMSO-*d*₆) δ 162.1, 158.7 (d, *J =* 253.1 Hz), 152.4 (d, *J* = 250.9 Hz), 149.7 (d, *J* = 9.2 Hz), 143.8 (d, *J* = 8.4 Hz), 132.1 (d, *J* = 11.4 Hz), 131.5 (d, *J* = 3.4 Hz), 129.6 (d, *J* = 15.8 Hz), 123, 5, 120.6 (d, *J* = 3.5 Hz), 119.7 (d, J = 3.8 Hz), 112.1 (d, *J* = 27.3 Hz), 111.9 (d, *J* = 24.7 Hz).

**3-fluoro-*N*-(3-fluoro-4-nitrophenyl)-4-nitrobenzamide (4j)**. Following the general method, **4j** was obtained as a solid (230 mg, 65 %). ¹H NMR (300 MHz, DMSO-*d*₆) δ 11.18 (s, 1H), 8.35 (t, *J* = 8.0 Hz, 1H), 8.25 (t, *J* = 8.9 Hz, 1H), 8.19 - 8.08 (m, 1H), 8.09 - 7.92 (m, 2H), 7.80 - 7.67 (m, 1H).¹³C NMR (75 MHz, DMSO-*d*₆) δ 163.6, 155.4 (d, *J* = 259.8 Hz), 154.2 (d, *J* = 262.3 Hz), 145.6 (d, *J=* 11.5 Hz), 140.5 (d, J = 7.5 Hz), 138.9 (d, *J* = 7.8 Hz), 131.9 (d, *J* = 7.0 Hz), 127.5, 126.7 (d, *J* = 2.7 Hz), 124.8 (d, *J* = 4.1 Hz), 118.1 (d, *J* = 22.7 Hz), 115.9 (d, *J* = 3.3 Hz), 108.3 (d, *J* = 26.1 Hz).

**2-fluoro-*N*-(2-fluoro-4-nitrophenyl)-4-nitrobenzamide (4k)**. Following the general method, **4k** was obtained as a whitish solid (270 mg, 77 %). ¹H NMR (300 MHz, DMSO-*d*₆) δ 11.07 (s, 1H), 8.45-8.12 (m, 5H), 8.00 (dd, *J* = 6.9, 8.5 Hz, 1H). ¹³C NMR (75 MHz, DMSO-*d*₆) δ 162.1, 158.7 (d, *J* = 253.3 Hz), 152.3 (*J* = 250.8 Hz), 154.0, 150.7, 149.7 (d, *J* = 9.1 Hz), 143.8 (d, *J* = 8.4 Hz), 132.1 (d, *J* = 11.6 Hz), 131.5 (d, *J =* 3.3 Hz), 129.6 (d, *J =* 15.7 Hz), 123.5, 120.6 (d, *J* = 3.3 Hz), 119.7 (d, *J* = 3.8 Hz), 112.1 (d, *J* = 27.5 Hz), 111.9 (d, *J* = 24.7 Hz).

**2-fluoro-*N*-(3-fluoro-4-nitrophenyl)-4-nitrobenzamide (4l)**. Following the general method, **4l** was obtained as a solid (272 mg, 77 %). ¹H NMR (300 MHz, DMSO-*d*₆) δ 11.46 (s, 1H), 8.33 (dd, *J* = 2.2, 9.7 Hz, 1H), 8.30 - 8.17 (m, 2H), 8.07 - 8.00 (m, 1H), 8.00 - 7.90 (m, 1H), 7.64 (dd, *J* = 2.3, 9.0 Hz, 1H). ¹³C NMR (75 MHz, DMSO-*d*₆) δ 162.1, 158.7 (d, *J* = 216.2 Hz), 153.8, 149.7 (d, *J* = 8.9 Hz), 145.2 (d, *J =* 11.4 Hz), 132.0 (d, *J* = 6.5 Hz), 131.3, 129.6 (d, *J =* 15.2 Hz), 127.7, 119.8 (d, *J =* 3.8 Hz), 115.5, 112.3 (d, *J* = 27.2 Hz), 107.9 (d, *J* = 26.1 Hz).

### 1.2. General method for the synthesis of the diamines 5b-l

SnCl₂.2H₂O (10 eq.) was added to a mixture of the dinitrobenzamide in EtOAc (20 mL) and a catalytic amount of concentrated HCl (20 drops). The reaction mixture is stirred overnight at 50 °C, achieving complete dissolution of the nitrobenzamide. The reaction crude is filtered over a layer of Celite, which is washed with a mixture of CH₂CL₂:MeOH (1:1) (250 mL). The filtrate is evaporated under high vacuum and the solid obtained is diluted in EtOAC, and it is basified (pH = 8) by adding a 10 % NaHCOs aqueous solution. The compound is extracted with EtOAc several times. A complete extraction of the aqueous phase is verified by TLC developed with ninhydrin. The organic phase is washed with a saturated sodium chloride solution, it is dried over MgSO₄, and the solvent was removed under high vacuum.

**5-amino-*No*-(5-aminopyridin-2-yl)picolinamide (5b).** The compound **4b** (1.5 g; 5.3 mmol) was hydrogenated in the presence of Pd-C 5 % (300 mg; 20 % w/w) with a Parr hydrogenator, obtaining 5b as a brown powder by recrystallisation in EtOAc (1.2 g; 99.8 %). ¹H NMR (400 MHz, DMSO-*d*₆) δ 9.80 (s, 1H), 7.96 (d, *J* = 2.7 Hz, 1H), 7.94 (d, *J =* 8.7 Hz, 1H), 7.82 (d, *J =* 8.7 Hz, 1H), 7.70 (d, *J* = 2.7 Hz, 1H), 7.03 (dd, *J* = 8.7, 2.7 Hz, 2H), 6.12 (s, 2H), 5.13 (s, 2H). ¹³C NMR (101 MHz, DMSO-*d*₆) δ 161.5, 148.0, 141.7, 141.3, 136.4, 134.4, 133.7, 123.1, 122.7, 119.4, 113.3. HPLC (UV) > 95 %. LRMS (ESI⁺): *m*/*z* 229.1 [M+H].

**4-amino-*N*-(4-amino-2-chlorophenyl)-3-chlorobenzamide (5c).** The compound **4c** (5.5 g; 15.4 mmol) and SnCl₂.2H₂O (35 g; 154 mmol) reacted following the general method, obtaining **5c** as a brown powder by recrystallisation in EtOAc (4.1 g; 90 %);¹H NMR (300 MHz, DMSO-*d*₆) δ 9.40 (s, 1H), 7.86 (d, *J* = 2.0 Hz, 1H), 7.67 (dd, *J* = 8.5, 2.0 Hz, 1H), 7.04 (d, *J* = 8.5 Hz, 1H), 6.81 (d, *J* = 8.5 Hz, 1H), 6.68 (d, *J* = 2.5 Hz, 1H), 6.51 (dd, *J* = 8.5, 2.5 Hz, 1H), 5.95 (br s, 2H), 5.33 (br s, 2H). ¹³C NMR (75 MHz, DMSO-*d*₆) δ 164.3, 148.3, 147.7, 130.7, 129.8, 128.8, 127.7, 123.0, 122.1, 116.1, 114.1, 113.4, 112.6. HPLC (UV) > 95 %. LRMS (ESI⁺): *m*/*z* 296.1 [M+H].

**4-amino-N-(4-amino-3-chlorophenyl)-3-chlorobenzamide (5d)**. The compound **4d** (2 g; 5.6 mmol) and SnCl₂.2H₂O (12.6 g; 562 mmol) reacted following the general method, obtaining **5d** as a yellowish powder by recrystallisation in EtOAc (4.1 g; 90 %). ¹H NMR (400 MHz, Methanol-*d*₄) δ 7.84 (d, *J* = 2.1 Hz, 1H), 7.64 (dd, *J* = 8.5, 2.1 Hz, 1H), 7.60 (d, *J* = 2.4 Hz, 1H), 7.27 (dd, *J* = 8.6, 2.4 Hz, 1H), 6.84 (d, *J* = 8.5 Hz, 1H), 6.83 (d, *J =* 8.6 Hz, 1H). ¹³C NMR (101 MHz, Methanol-*d*₄) δ 167.4, 149.2, 142.0, 131.1, 130.1, 128.5, 124.3, 123.8, 122.7, 119.8, 118.7, 117.1, 115.5. HPLC (UV) > 95 %. LRMS (ESI⁺): *m*/*z* 296.1 [M+H].

**4-amino-N-(4-amino-2-chlorophenyl)-2-chlorobenzamide (5e).** The compound **4e** (1.4 g; 4.0 mmol) and SnCl₂.2H₂O (9 g; 40 mmol) reacted following the general method, obtaining **5e** as a brown powder by recrystallisation in EtOAc (693 mg; 59 %). ¹H NMR (400 MHz, DMSO-*d*₆) δ 9.25 (s, 1H), 7.33 (d, *J* = 8.2 Hz, 1H), 7.16 (d, *J =* 8.4 Hz, 1H), 6.66 (d, *J* = 2.6 Hz, 1H), 6.62 (d, *J* = 2.2 Hz, 1H), 6.56 - 6.47 (m, 2H), 5.75 (s, 2H), 5.32 (s, 2H). ¹³C NMR (126 MHz, DMSO-*d*₆) δ 165.3, 151.5, 148.0, 131.5, 130.8, 129.7, 128.8, 122.9, 122.2, 113.8, 113.4, 112.6, 111.6. HPLC (UV) > 95 %. LRMS (ESI⁺): *m*/*z* 296.1 [M+H].

**4-amino-*N*-(4-amino-3-chlorophenyl)-2-chlorobenzamide (5f).** The compound **4f** (1 g; 2.5 mmol) and SnCl₂.2H₂O (4.75 g; 25 mmol) reacted following the general method, obtaining **5f** as a yellowish powder by recrystallisation in CHCl₃ (520 mg; 71 %). ¹H NMR (300 MHz, DMSO-*d*₆) δ 9.83 (s, 1H), 7.68 (d, *J* = 2.4 Hz, 1H), 7.28 (dd, *J* = 8.8, 2.4 Hz, 1H), 7.23 (d, *J* = 8.4 Hz, 1H), 6.75 (d, *J=* 8.8 Hz, 1H), 6.63 (d, *J* = 2.1 Hz, 1H), 6.53 (dd, *J* = 8.4, 2.1 Hz, 1H), 5.74 (s, 2H), 5.12 (s, 2H). HPLC (UV) > 95 %. LRMS (ESI⁺): *m*/*z* 296.1 [M+H].

**4-amino-N-(4-amino-3-isopropoxypheyil)-2-isopropoxybenzamide (5g).** The compound **4g** (324 mg: 0.8 mmol) was hydrogenated in the presence of Pd-C 5 % (65 mg) with a Parr hydrogenator, obtaining **5g** as a brown solid (253 mg, 92.4 %) by recrystallisation in EtOAc/Hexane. ¹H NMR (300 MHz, DMSO-*d*₆) δ 9.51 (s, 1H), 7.87 (d, *J* = 8.6 Hz, 1H), 7.72 (d, *J* = 8.5 Hz, 1H), 6.32 (d, *J* = 2.3 Hz, 1H), 6.30 (d, *J* = 2.0 Hz, 1H), 6.23 (dd, *J* = 8.5, 2.0 Hz, 1H), 6.12 (dd, *J =* 8.6, 2.3 Hz, 1H), 5.72 (s, 2H), 4.86 (s, 2H), 4.65 (d, *J* = 6.0 Hz, 1H), 4.49 (d, *J* = 6.0 Hz, 1H), 1.39 (d, *J* = 6.0 Hz, 6H), 1.30 (d, *J* = 6.0 Hz, 6H). ¹³C NMR (75 MHz, DMSO-*d*₆) δ 162.3, 157.1, 153.3, 148.0, 145.2, 132.8, 122.6, 118.4, 110.3, 106.8, 105.6, 100.1, 98.4, 71.6, 70.6, 22.2, 22.0.

**4-amino-N-(4-amino-2-fluorophenyl)-3-fluorobenzamide (5i).** The compound **4i** (476 mg, 1.47 mmol) was hydrogenated in the presence of Pd-C 5 % (86 mg) with a Parr hydrogenator, obtaining **5i** as a greyish solid (230 mg, 59.4 %) by recrystallisation in EtOAc/MeOH (98/2). ¹H NMR (300 MHz, DMSO-*d*₆) δ 9.32 (s, 1H), 7.59 (m, 2H), 7.01 (t, *J* = 8.7 Hz, 1H), 6.77 (t, *J* = 8.7 Hz, 1H), 6.38 (m, 2H), 5.76 (s, 2H), 5.30 (s, 2H).¹³C NMR (75 MHz, DMSO-*d*₆) δ 164.3, 157.4 (d, *J* = 243.1 Hz), 149.4 (d, *J* = 236.9 Hz), 148.5 (d, *J* = 11.0 Hz), 139.9 (d, *J =* 13.0 Hz), 128.8 (d, *J* = 3.5 Hz), 124.7, 121.1 (d, *J* = 5.5 Hz), 114.7 (d, *J* = 4.9 Hz), 114.3 (d, *J=* 19.6 Hz), 113.4 (d, *J=* 13.5 Hz), 109.3, 100.4 (d, *J* = 23.0 Hz).

**4-amino-N-(4-amino-3-fluorophenyl)-3-fluorobenzamide (5j).** The compound **4j** (177 mg, 0.55 mmol) was hydrogenated in the presence of Pd-C 5 % with a Parr hydrogenator (H₂, 24 Psi) for 2.5 h, obtaining 5j as a greyish solid by recrystallisation in EtOAc. ¹H NMR (300 MHz, DMSO-*d*₆) δ 9.67 (s, 1H), 7.74 - 7.42 (m, 3H), 7.21 - 7.16 (m, 1H), 6.88 - 6.65 (m, 2H), 5.78 (s, 2H), 4.90 (s, 2H). ¹³C NMR (75 MHz, DMSO-*d*₆) δ 163.7, 149.7 (d, *J* = 235.8 Hz), 148.9 (d, *J* = 236.1 Hz), 139.9 (d, *J* = 13.1 Hz), 132.1 (d, *J* = 13.0 Hz), 128.9 (d, *J =* 9.5 Hz), 124.7, 121.6 (d, *J* = 5.5 Hz), 116.8 (d, *J =* 3.1 Hz), 115.8 (d, *J* = 5.4 Hz), 114.7 (d, *J* = 5.0 Hz), 114.2 (d, *J* = 19.3 Hz), 108.2 (d, *J* = 23.0 Hz).

**4-amino-*N*-(4-amino-2-fluorophenyl)-2-fluorobenzamide (5k)**. The compound **4k** (200 mg, 0.62 mmol) was hydrogenated in the presence of Pd-C 5 % (60 mg) with a Parr hydrogenator (H₂, 28 Psi) for 2 h, obtaining **5k** as a greyish solid by recrystallisation in EtOAc. ¹H NMR (300 MHz, DMSO-*d*₆) δ 8.82 (d, *J* = 7.3 Hz, 1H), 7.49 (t, *J* = 8.8 Hz, 1H), 7.26 (t, *J* = 8.8 Hz, 1H), 6.46 - 6.27 (m, 4H), 6.00 (s, 2H), 5.28 (s, 2H). ¹³C NMR (75 MHz, DMSO-*d*₆) δ 162.3, 161.5 (d, *J* = 245.5 Hz), 156.2 (d, *J* = 241.9 Hz), 153.7 (d, *J =* 12.7 Hz), 147.9 (d, *J =* 10.8 Hz), 131.9 (d, *J* = 4.8 Hz), 127.1, 113.9 (d, *J =* 12.7 Hz), 109.5, 109.2 (d, *J* = 2.1 Hz), 108.7 (d, *J* = 12.8 Hz), 100.3 (d, *J* = 22.8 Hz), 99.2 (d, *J* = 26.6 Hz).

**4-amino-*N*-(4-amino-3-fluorophenyl)-2-fluorobenzamide (5l)**. The compound **4l** (155 mg, 0.48 mmol) was hydrogenated in the presence of Pd-C 5 % (50 mg) with a Parr hydrogenator (H₂, 23 Psi) for 2 h, obtaining **5l** as a greyish solid (20 mg, 16 %) by recrystallisation in EtOAc. ¹H NMR (300 MHz, DMSO-*d*₆) δ 9.77 - 9.27 (m, 1H), 7.60 - 7.32 (m, 2H), 7.22 - 7.04 (m, 1H), 6.70 (t, *J* = 9.6 Hz, 1H), 6.48 - 6.24 (m, 2H), 5.95 (s, 2H), 4.89 (s, 2H). ¹³C NMR (75 MHz, DMSO-*d*₆) δ 162.2, 161.0 (d, *J* = 246.6 Hz), 153.4 (d, *J =* 12.5 Hz), 149.7 (d, *J* = 235.7 Hz), 132.1 (d, *J =* 13.1 Hz), 131.4 (d, *J* = 4.9 Hz), 128.8 (d, *J* = 9.6 Hz), 116.4 (d, *J* = 2.9 Hz), 115.8 (d, *J* = 5.4 Hz), 110.2 (d, *J* = 12.6 Hz), 109.4, 107.8 (d, *J* = 23.2 Hz), 99.3 (d, *J* = 26.0 Hz).

**1,3-bis(4-aminophenyl)urea (10).** 1,3-bis(4-nitrophenyl)urea (2.4 g; 8.0 mmol) was hydrogenated in the presence of Pd-C 5 % (213 mg) with a Parr hydrogenator, obtaining **10** as a slightly violet powder by recrystallisation in MeOH/DMF (520 mg; 27 %). ¹H NMR (300 MHz, DMSO-*d*₆) δ 7.91 (s, 2H), 7.04 (d, *J* = 8.1 Hz, 4H), 6.49 (d, *J* = 8.1 Hz, 4H), 4.71 (s, 4H). HPLC (UV) > 95 %. LRMS (ESI⁺): *m*/*z* 243.3 [M+H].

### 1.3. General method for the synthesis of isothiocyanates (6a-g, 11, 12)

The reaction is carried out in KIMAX tubes. A solution of diamine (1 eq.) and thiophosgene (2.5 eq.) in an Et₂O:H₂O mixture (3:1) (2 mL) is stirred at room temperature overnight. After stirring a solid appears at the bottom of the tube. The precipitated solid is filtered on a plate and washed with distilled H₂O (100 mL). Purification is carried out in a column with Et₂O:EtOAc. The product is dried at 40 °C, obtaining a solid.

**4-isothiocyanato-*N*-(4-isothiocyanatophenyl)benzamide (6a).** The 4-amino-*N*-(4-aminophenyl)benzamide **5a** (3.4 g, 15 mmol), commercially available, reacted with thiophosgene (2.5 mL, 33 mmol) following the general method, obtaining **6a** as a greyish solid (92 %). ¹H NMR (300 MHz, Chloroform-d) δ 7.88 (s 1H, NH), 7.85 (d, *J* = 8.7, 2H), 7.63 (d, *J* = 8.8, 2H), 7.31 (d, *J* = 8.7, 2H), 7.22 (d, *J* = 8.8, 2H). ¹³C NMR (75 MHz, CDCl₃) δ 164.7, 138.6, 137.1, 135.9, 135.5, 133.2, 129.0, 127.8, 127.0, 126.5, 121.5. Mp. 199-200 °C. HPLC (UV) > 95 %. LRMS (ESI⁺) *m*/*z* = 312.3 (M+H).

**5-isothiocyanato-N-(5-isothiocyanatopyridin-2-yl)picolinamide (6b). 5b** (74.5 mg, 0.32 eq.) reacted with thiophosgene (62 µL, 0.81 eq.) following the general method, obtaining **6b** as a brown solid. The compound was purified by chromatography on silica eluting with petroleum etherEtOAc (100:0 -> 50:50). ¹H NMR (300 MHz, DMSO-*d*₆) δ 10.43 (s, 1H), 8.81 (s, 1H), 8.63 - 8.43 (m, 1H), 8.37 - 8.12 (m, 3H), 8.00 (m, 1H). ¹³C NMR (75 MHz, DMSO-*d*₆) δ 181.4, 172.1, 165.0, 162.2, 153.8, 146.3, 139.9, 136.2, 129.7, 126.3, 124.6, 123.6. HPLC (UV) > 95 %. LRMS (ESI⁺) *m*/*z* = 314.3 (M+H).

**3-chloro-*N*-(2-chloro-4-isothiocyanatophenyl)-4-isothiocyanatobenzamide (6c). 5c** (100 mg, 0.34 mmol) reacted with thiophosgene (65 µL, 0.85 mmol) following the general method, obtaining **6c** as a brown solid (44 %). ¹H NMR (300 MHz, Chloroform-d) δ 8.53 (d, *J* = 8.9 Hz, 1H), 8.31 (s, 1H), 7.98 (s, 1H), 7.74 (d, *J* = 8.3 Hz, 1H), 7.36 (dd, *J* = 8.3, 1.1 Hz, 1H), 7.21 (dd, *J* = 8.9, 1.3 Hz, 1H). HPLC (UV) > 95 %. LRMS (ESI⁺) *m*/*z* = 377.8 (M-H).

**3-chloro-*N*-(3-chloro-4-isothiocyanatophenyl)-4-isothiocyanatobenzamide (6d). 5d** (150 mg, 0.51 mmol) reacted with thiophosgene (147 mg, 0.1 mL, 1.28 mmol) following the general method, obtaining **6d** as a whitish solid (160 mg, 83 %). ¹H NMR (300 MHz, DMSO-*d*₆) δ 10.63 (s, 1H), 8.15 (d, *J* = 2.0 Hz, 1H), 8.08 (d, *J* = 2.4 Hz, 1H), 7.94 (dd, *J* = 8.4, 2.0 Hz, 1H), 7.73 (dd, *J* = 8.8, 2.4 Hz, 1H), 7.67 (d, *J* = 8.4 Hz, 1H), 7.51 (d, *J =* 8.8 Hz, 1H). ¹³C NMR (75 MHz, DMSO-*d*₆) δ 163.2, 139.1, 138.7, 136.7, 134.0, 131.4, 130.5, 130.4, 129.2, 128.0, 127.5, 127.3, 123.3, 120.7, 119.7. HPLC (UV) > 95 %. LRMS (ES⁻) *m*/*z* = 377.7 (M-H).

**2-chloro-*N*-(2-chloro-4-isothiocyanatophenyl)-4-isothiocyanatobenzamide (6e). 5e** (100 mg, 0.34 mmol) reacted with thiophosgene (98 mg, 65 µL, 0.85 mmol) following the general method. The product was purified on silica chromatography eluting with petroleum ether: EtOAc (100:0 → 25:75), obtaining 6e as a whitish solid (85 mg, 66 %). ¹H NMR (300 MHz, DMSO-*d₆*) δ 10.41 (s, 1H), 7.79 (d, *J=* 9.2 Hz, 1H), 7.75 (d, *J* = 2.0 Hz, 1H), 7.74 (d, *J* = 2.4 Hz, 1H), 7.68 (d, *J* = 8.2 Hz, 1H), 7.54 (dd, *J* = 8.2, 2.0 Hz, 1H), 7.49 (dd, *J* = 9.0, 2.4 Hz, 1H). HPLC (UV) > 95 %. LRMS (ESI⁻) *m*/*z* = 377.8 (M-H).

**2-chloro-*N*-(3-chloro-4-isothiocyanatophenyl)-4-isothiocyanatobenzamide (6f). 5f** (310 mg, 1.05 mmol) reacted with thiophosgene (303 mg, 0.2 mL, 2.63 mmol) following the general method, obtaining **6f** as a whitish solid (94 mg, 24 %). ¹H NMR (300 MHz, Chloroform-d) δ 8.15 (s, 1H), 7.86 (d, *J* = 2.3 Hz, 1H), 7.74 (d, *J=* 8.2 Hz, 1H), 7.45 (dd, *J* = 8.6, 2.3 Hz, 1H), 7.28 (d, *J* = 2.0 Hz, 1H), 7.22 (d, *J* = 8.6 Hz, 1 H), 7.20 (dd, *J* = 8.2, 2.0 Hz, 1 H). ¹³C NMR (75 MHz, Chloroform-d) δ 163.3, 140.1, 139.1, 136.8, 135.4, 132.6, 132.6, 132.0, 131.8, 127.4, 127.1, 126.3, 124.8, 121.5, 119.1. HPLC (UV) > 95 %. LRMS (ESI⁻) *m*/*z* = 377.8 (M-H)

**1,2-bis(4-isothiocyanatophenyl)ethane (11).** 4,4'-diaminobibenzyl **9** (1,28 g, 6 mmol) reacted with thiophosgene (1.73 g: 1.15 mL, 15 mmol) following the general method. The product was purified by chromatography on silica eluting with n-hexane: EtOAc (100→0 to 80→20), obtaining **11** as a whitish solid (1.4 g, 79 %). ¹H NMR (400 MHz, Chloroform-d) δ 7.12 (d, *J* = 8.6 Hz, 4H), 7.07 (d, *J* = 8.6 Hz, 4H), 2.89 (s, 4H). HPLC (UV) > 95 %. LRMS (ESI⁺) *m*/*z* = 297.3 (M+H).

**1,3-bis(4-isothiocyanatophenyl)urea (12). 10** (201 mg, 0.83 mmol) reacted with thiophosgene (0.16 mL, 2.08 mmol) following the general method, obtaining **12** as a whitish solid (174 mg: 64 %). ¹H NMR (400 MHz, DMSO-*d*₆) δ 9.03 (s, 2H), 7.52 (d, *J* = 8.9 Hz, 4H), 7.37 (d, *J* = 8.9 Hz, 4H).

¹³C NMR (101 MHz, DMSO-*d*₆) δ 152.1, 139.2, 132.2, 126.7, 123.2, 119.0. HPLC (UV) > 95 %. LRMS (ES⁺) *m*/*z* = 314.3 (M+H).

### 1.4. General method for the synthesis of bisbenzimidazoles (1a-f, 13, 14)

In a KIMAX tube, a solution of the isothiocyanate (1 eq.) and o-phenylenediamine (2.2 eq.) is prepared in anhydrous DMF (4 mL) and stirred at 0 °C (ice bath) until complete formation of the thiourea intermediate (verified by TLC and HPLC-MS). The EDC hydrochloride (2.5 eq.) is then added in a single step and the reaction mixture is stirred at 60 °C for 24 hours. After said time has elapsed, pieces of ice are added to the reaction crude and the tube is vigorously stirred. The precipitated solid is purified by circular chromatography (Chromatotron) using CH₂CL₂:MeOH (9:1; 8:2; 7:3; 6:4; 1:1) as an elution system.

**4-((1*H*-benzo[*d*]imidazol-2(3*H*)-ylidene)amino)-N-(4-((1*H*-benzo[*d*]imidazol-2(3*H*)-ylidene)amino)phenyl)benzamide (1a).** A solution of **6a** (200 mg, 0.64 mmol) and o-phenylenediamine (151 mg, 1.4 mmol) in anhydrous DMF (10 mL) was stirred at room temperature for 2 h until the reagents were consumed. Iodine (405 mg, 1.6 mmol) was added to the reaction mixture, followed by potassium carbonate (221 mg, 1.6 mmol). The reaction mixture was stirred overnight and the reaction was stopped by adding a 5 % Na₂S₂O₃ aqueous solution (3.5 mL). Brine (50 mL) was added and the aqueous phase was extracted with CH₂Cl₂ (3 × 30 mL). The organic phase was dried and the solvent was evaporated, obtaining a yellowish oil. The product was purified by chromatography on silica (Hexane:EtOAc, 100:0->40:60), obtaining a brown solid. ¹H NMR (300 MHz, Methanol-*d*₄) δ 8.20 (d, *J =* 8.6 Hz, 2H), 7.98 (d, *J =* 8.8 Hz, 2H), 7.64 (d, *J =* 8.8 Hz, 2H), 7.58 - 7.43 (m, 6H), 7.40 - 7.28 (m, 4H). HPLC (UV) > 95 %. LRMS (ESI⁺) *m*/*z* = 459.2 [M+H].

**5-((1*H*-benzo[*d*]imidazol-2(3*H*)-ylidene)amino)-*N*-(5-((1*H*-benzo[*d*]imidazol-2(3*H*)-ylidene)amino)pyridin-2-yl)picolinamide (1b).** The reaction was carried out following the general method using **6b** (20 mg, 64 pmol), o-phenylenediamine (15 mg, 140 µmol), DMF (4 mL) and EDC hydrochloride (30.6 mg, 160 µmol) with 12 h of stirring at 60 °C. After this time, ice was added to the reaction mixture to precipitate the product. The solid was isolated by filtration, washed with cold water and dried, obtaining a brown solid.¹H NMR (300 MHz, DMSO-*d*₆) δ 11.32 (s, 1H), 11.07 (s, 1H), 10.26 (s, 1H), 10.19 (s, 1H), 9.60 (s, 1H), 8.96 (d, *J* = 2.5 Hz, 1H), 8.77 (d, *J* = 2.6 Hz, 1H), 8.66 (dd, *J* = 8.5, 2.2 Hz, 1H), 8.33 (dd, *J* = 9.0, 2.6 Hz, 1H), 8.26 (d, *J* = 8.9 Hz, 1H), 8.19 (d, *J* = 8.7 Hz, 1H), 7.52 -7.23 (m, 4H), 7.11 - 6.93 (m, 4H). ¹³C NMR (75 MHz, DMSO-*d*₆) δ 161.5, 150.3, 149.2, 144.5, 142.9, 142.5, 140.8, 140.4, 137.5, 137.4, 134.4, 132.9, 132.7, 126.7, 123.7, 123.0, 120.9, 120.6, 119.9, 116.5, 116.0, 113.0, 110.0, 109.5. HPLC (UV) > 95 %. LRMS (ESI⁺) *m*/*z* = 462.2 [M+H].

**4-((1*H*-benzo[*d*]imidazol-2(3*H*)-ylidene)amino)-*N*-(4-((1*H*-benzo[*d*]imidazol-2(3*H*)-ylidene)amino)-2-chlorophenyl)-3-chlorobenzamide (1c).** The reaction was carried out following the general method using **6c** (125 mg, 0.33 mmol), o-phenylenediamine (78 mg, 0.72 mmol), DMF (4 mL) and EDC hydrochloride (157 mg, 0.82 mmol) with 12 h of stirring at 60 °C. The product was purified by circular chromatography on silica (CH₂Cl₂:MeOH, 100:0→70:30), obtaining a yellowish solid (75 mg, 43 %). ¹H NMR (500 MHz, DMSO-*d*₆) δ 10.04 (s, 1H), 9.04 (t, *J* = 5.7 Hz, 1H), 8.86 (d, *J =* 8.9 Hz, 1H), 8.17 (d, *J* = 2.1 Hz, 1H), 8.12 (d, *J* = 2.5 Hz, 1H), 8.05 (dd, *J* = 8.7, 2.1 Hz, 1H), 7.61 (dd, *J =* 8.6, 2.5 Hz, 1H), 7.50 (d, *J* = 8.7 Hz, 1H), 7.45 (dd, *J* = 5.9, 3.2 Hz, 1H), 7.38 (td, *J* = 6.2, 3.2 Hz, 3H), 7.21 (dd, *J* = 5.9, 3.2 Hz, 1H), 7.09 (td, *J* = 6.2, 3.2 Hz, 4H), 3.46 - 3.40 (m, 2H). ¹³C NMR (126 MHz, DMSO-*d*₆) δ 163.9, 150.0, 149.5, 149.3, 139.6, 139.1, 130.4, 129.9, 129.4, 128.8, 128.5, 127.7, 127.4, 123.0, 121.0, 120.7, 118.6, 118.4, 117.2, 111.2. HPLC (UV) > 95 %. LRMS (ESI⁺) *m*/*z* = 528.2 [M+H].

**4-((1*H*-benzo[*d*]imidazol-2(3*H*)-ylidene)amino)-*N*-(4-((1*H*-benzo[*d*]imidazol-2(3*H*)-ylidene)amino)-3-chlorophenyl)-3-chlorobenzamide (1d).** The reaction was carried out following the general method using **6d** (102 mg, 0.27 mmol), o-phenylenediamine (64 mg, 0.60 mmol), DMF (4 mL) and EDC hydrochloride (131 mg, 0.68 mmol) with 48 h of stirring at 60 °C. The product was purified by circular chromatography on silica (CH₂Cl₂:MeOH, 100:0→70:30), obtaining a yellowish solid (21 mg; 15 %).¹H NMR (400 MHz, DMSO-*d*₆) δ 10.93 (br s, 3H), 9.50 (br s, 2H), 9.23 (br s, 2H), 8.85 (d, *J* = 9.8 Hz, 2H), 8.72 (d, *J* = 9.8 Hz, 2H), 8.59 - 8.30 (m, 6H), 3.16 (br s, 2H). ¹³C NMR (101 MHz, DMSO-*d*₆) δ 161.1, 154.2, 152.1, 146.1, 144.6, 143.8, 140.7, 134.4, 133.6, 123.4, 112.9. HPLC (UV) > 95 %. LRMS (ES⁺) *m*/*z* 528.1 [M+H].

**4-((1*H*-benzo[*d*]imidazol-2(3*H*)-ylidene)amino)-*N*-(4-((1*H*-benzo[*d*]imidazol-2(3*H*)-ylidene)amino)-2-chlorophenyl)-2-chlorobenzamide (1e).** The reaction was carried out following the general method using **6e** (50 mg, 0.13 mmol), o-phenylenediamine (31 mg, 0.29 mmol), DMF (4 mL) and EDC hydrochloride (63 mg, 0.33 mmol) with 48 h of stirring at 60 °C. The product was purified by circular chromatography on silica (CH₂Cl₂:MeOH-NH_{3(saturated)}, 100:0→80:20), obtaining a yellowish solid (20 mg; 17 %). ¹H NMR (400 MHz, DMSO-*d*₆) δ 11.17 (s, 1H), 11.06 (s, 1H), 9.92 (s, 1H), 9.84 (s, 1H), 9.70 (s, 1H), 8.23 (d, *J* = 2.4 Hz, 1H), 8.21 (d, *J* = 2.4 Hz, 1H), 7.69 (dd, *J* = 2.2, 8.1 Hz, 1H), 7.59 - 7.53 (m, 3H), 7.43 (d, *J* = 7.1 Hz, 1H), 7.40 (d, *J =* 7.1 Hz, 1H), 7.32 (t, *J* = 8.7 Hz, 2H), 7.04 (m, 4H). ¹³C NMR (101 MHz, DMSO-*d₆*) δ 165.2, 150.0, 149.5, 143.2, 142.8, 142.6, 139.9, 132.6, 132.6, 131.0, 130.1, 129.2, 128.2, 127.5, 127.2, 120.7, 120.6, 120.3, 120.0, 117.0, 117.0, 116.3, 116.1, 115.9, 115.0, 109.8, 109.6. HPLC (UV) > 95 %. LRMS (ESI⁺) *m*/*z* = 528.2 [M+H].

**4-((1*H*-benzo[d]imidazol-2(3*H*)-ylidene)amino)-N-(4-((1*H*-benzo[*d*]imidazol-2(3*H*)-ylidene)amino)-3-chlorophenyl)-2-clorobenzamide (1f).** The reaction was carried out following the general method using **6f** (83 mg, 0.22 mmol), o-phenylenediamine (52 mg, 0.48 mmol), DMF (4 mL) and EDC hydrochloride (106 mg, 0.55 mmol) with 72 h of stirring at 60 °C. The product was purified by circular chromatography on silica (CH₂Cl₂: MeOH-NH_{3(saturated)}, 100:0→90:10), obtaining a yellowish solid (68 mg; 59 %). ¹H NMR (500 MHz, Methanol-*d*₄) δ 8.07 (d, *J =* 8.8 Hz, 1H), 8.02 (d, *J =* 2.4 Hz, 1H), 7.85 (d, *J =* 2.1 Hz, 1H), 7.57 (dd, *J* = 8.8, 2.4 Hz, 1H), 7.56 (d, *J* = 8.5 Hz, 1H), 7.53 (dd, *J* = 8.5, 2.1 Hz, 1H), 7.43 - 7.29 (m, 4H), 7.12 (m, 2H), 7.07 (m, 2H). ¹³C NMR (126 MHz, Methanol-*d*₄) δ 168.0, 152.4, 151.1, 144.9, 135.9, 134.5, 133.2, 131.0, 129.9, 125.5, 123.0, 122.5, 122.4, 122.2, 120.8, 119.1, 116.7. HPLC (UV) 94.2 %. HRMS (ESI⁺) *m*/*z* = 528.2 [M+H].

**4,4'-(ethane-1,2-diyl)bis(*N*-(1*H*-benzo[*d*]imidazol-2(3*H*)-ylidene)aniline) (13).** The reaction was carried out following the general method using **11** (225 mg; 0.76 mmol), o-phenylenediamine (180 mg; 1.67 mmol), DMF (3 mL) and EDC hydrochloride. The product was purified by circular chromatography on silica (CH₂Cl₂:MeOH, 100:0 → 92:8), obtaining a whitish solid (182 mg, 54 %). ¹H NMR (400 MHz, DMSO-*d*₆) δ 9.29 (s, 2H), 7.63 (d, *J* = 8.5 Hz, 4H), 7.29 (dd, *J* = 5.8, 3.2 Hz, 4H), 7.16 (d, *J* = 8.5 Hz, 4H), 6.97 (dd, *J=* 5.8, 3.2 Hz, 4H), 2.83 (s, 4H). ¹³C NMR (101 MHz, DMSO-*d*₆) δ 162.4, 150.8, 138.7, 133.8, 128.7, 120.0, 117.2, 36.7. HPLC (UV) > 95 %. LRMS (ESI⁺) *m*/*z* = 445.3 [M+H].

**1,3-bis(4-((1*H*-benzo[*d*]imidazol-2(3*H*)-ylidene)amino)phenyl)urea (14).** The reaction was carried out following the general method using **12** (103 mg, 0.32 mmol), o-phenylenediamine (756 mg, 0.7 mmol), DMF (1 mL) and EDC hydrochloride (154 mg, 0.8 mmol) with 48 h of stirring at 60 °C. The product was purified by circular chromatography on silica (CH₂Cl₂: MeOH-NH_{3(saturated)} 98.75:1.25 -> 80:20), obtaining a yellowish solid (38 mg: 25 %). ¹H NMR (400 MHz, DMSO-*d*₆) δ 10.86 (br s, 2H), 9.25 (br s, 2H), 8.47 (s, 2H), 7.65 (d, *J* = 8.9 Hz, 4H), 7.39 (d, *J* = 8.9 Hz, 4H), 7.28 (dd, *J* = 5.8, 3.3 Hz, 4H), 6.97 (dd, *J* = 5.8, 3.3 Hz, 4H). ¹³C NMR (101 MHz, DMSO-*d*₆) δ 171.4, 152.9, 152.9, 151.0, 135.3, 133.3, 119.8, 119.1, 117.9. HPLC (UV) > 95 %. LRMS (ESI⁺) *m*/*z* 475.5 [M+H].

### 1.7. Preparation of methyl (Z)-N-(tert-butoxycarbonyl)pyridine-2-carbimidothioate (7)

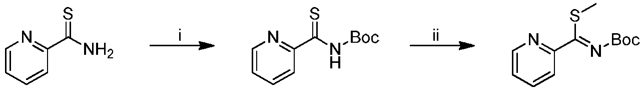

### Synthesis of methyl (Z)-N-(tert-butoxycarbonyl)pyridine-2-carbimidothioate (7).

Reagents and conditions: (i) NaH, DMF, di-tert-butyldicarbonate, 0 °C → 25 °C; (ii) NaH, DMF, CH₃I, 0 °C → 25 °C.

A solution of NaH (2.2 eq) in anhydrous DMF is stirred at 0 °C (ice bath). Then, the pyridine-2-carbothioamide (1 eq) is added and the mixture is stirred for 5 minutes, followed by the slow addition of di-tert-butyl dicarbonate (2.2 eq) and stirring at room temperature for 2 hours. The reaction crude is neutralised with a saturated NaHCOs solution. The organic phase is extracted with EtOAc, washed with a saturated NaCl solution, dried over MgSO₄. The organic phase is concentrated under vacuum to obtain the Boc-protected intermediate as a yellow oil.

The Boc-protected intermediate (19 g, 79.7 mmol, 1 eq), is added to a NaH solution (4.0 g, 167.4 mmol, 2.1 eq) in anhydrous DMF (300 mL) stirred at 0 °C (ice bath), after 15 minutes of stirring, methyl iodide (22.6 g, 159.5 mmol, 2 eq) is added stirring at room temperature. The reaction crude oil is neutralised with a saturated NaHCOs solution (200 mL). The organic phase is extracted with EtOAc (3 × 200 mL), washed with brine, dried over MgSO₄. The organic phase is concentrated under vacuum. The product is purified by silica column chromatography eluting with a mixture of hexane:EtOAC (100:0 → 90:10), obtaining a yellowish solid. The product dissolved in CH₃CN/H₂O was lyophilised, obtaining a whitish solid (10.9 g, 54.3 %). ¹H NMR (400 MHz, DMSO-*d*₆) δ 8.64 (dd, *J* = 4.7, 1.6 Hz, 1H), 7.97 (td, *J* = 7.7, 1.6 Hz, 1H), 7.73 (dd, *J* = 7.7, 1.1 Hz, 1H), 7.56 (ddd, *J* = 7.7, 4.7, 1.1 Hz, 1H), 2.43 (s, 3H), 1.38 (s, 9H). ¹³C NMR (101 MHz, DMSO-*d*₆) δ 169.4, 159.3, 150.9, 149.1, 137.8, 126.3, 121.9, 80.9, 27.5, 13.6.

### 1.8. General method for the synthesis of Boc-protected 2-arylimidamides (2a-l)

A microwave tube was loaded with methyl (*Z*)-*N*-tert-butoxycarbonylpyridine-2-carbimidethioate **7** (4 eq.), diamine **5a-l** (1 eq.) and mercury chloride (II) (4 eq.). The tube is sealed and anhydrous CH₂Cl₂ (5 mL) is added, followed by the addition of anhydrous triethylamine (7 eq.). The reaction mixture is irradiated for 1 h at 50 °C. The reaction mixture is diluted in CH₂Cl₂ and is filtered over Celite, using a mixture of CH₂Cl₂:MeOH (50 mL). The solvent is stirred under vacuum. The pure compound is obtained by chromatography.

***tert*-butyl ((*E*)-((6-((5-((*E*)-*N*'*-*(*tert*-butoxycarbonyl)picolinimidamido)pyridin-2-yl)carbamoyl)pyridin-3-yl)amino)(pyridin-2-yl)methylene)carbamate (2b)**. The reaction was carried out with **5b** (99 mg; 0.43 mmol), 7 (436 mg; 1.73 mmol), HgCl₂ (469 mg; 1.73 mmol), Et₃N (1.75 mL; 1.73 mmol) and anhydrous dichloromethane (2 mL) following the general method. The product was purified by flash chromatography on silica eluting with Hexane/EtOAc (100:0→80:20), obtaining a yellowish solid (97 mg; 36 %). ¹H NMR (300 MHz, DMSO-*d*₆) δ 10.49 (br s, 8H), 9.78 (s, 1H), 8.97 (br s, 2H), 8.03 - 7.88 (m, 2H), 7.81 (d, *J* = 8.5 Hz, 1H), 7.70 (d, *J* = 2.8 Hz, 1H), 7.03 (dd, *J* = 8.7, 2.5 Hz, 2H), 6.16 (s, 1H), 5.14 (s, 1H), 1.20 (s, 18H). ¹³C NMR (126 MHz, DMSO-*d*₆) δ 161.5, 148.1, 141.7, 141.3, 136.3, 134.4, 133.7, 123.1, 122.6, 119.3, 113.3, 8.4. HPLC (UV) > 95 %. LRMS (ESI⁺): *m*/*z* = 638.4 [M+H].

***tert*-butyl ((*E*)-((4-((4-((*E*)-*N*'-(*tert*-butoxycarbonyl)picolinimidamido)-2-chlorophenyl)carbamoyl)-2-chlorophenyl)amino)(pyridin-2-yl)methylene)carbamate (2c).** The reaction was carried out with **5c** (150 mg, 0.5 mmol), **7** (515 mg, 2 mmol), HgCl₂ (554 mg, 2 mmol), Et₃N (0.3 mL, 2 mmol) and anhydrous dichloromethane (2 mL) following the general method. The product was purified by reverse phase column chromatography (C18) with a 12 g cartridge using H₂O:CH₃CN (100:0 → 0:100), obtaining a yellowish solid (159 mg, 44 %). ¹H NMR (500 MHz, DMSO-*d*₆) δ 10.04 (s, 1H), 9.99 (br s, 2H), 8.70 (ddd, *J* = 4.8, 1.7, 1.0 Hz, 2H), 8.62 (ddd, *J =* 3.6, 1.7, 1.0 Hz, 1H), 8.08 - 7.98 (m, 3H), 7.96 - 7.83 (m, 1H), 7.74 - 7.67 (m, 3H), 7.59 (ddd, *J* = 7.7, 4.8, 1.0 Hz, 2H), 7.50 (d, *J* = 8.7 Hz, 2H), 1.23 (br s, 18H). ¹³C NMR (126 MHz, DMSO-*d*₆) δ 163.9, 160.1, 156.3, 151.4, 151.1, 149.3, 149.1, 148.5, 138.5, 137.2, 130.2, 129.6, 128.9, 128.8, 127.0, 125.6, 125.0, 123.2, 123.1, 120.8, 119.5, 80.4, 79.0, 27.6, 27.5. HPLC (UV) > 92 %. LRMS (ESI⁺): *m*/*z* 704.3 [M+H].

***tert*-butyl ((*E*)-((4-((4-((*AND*)-*N*'-(tert-butoxycarbonyl)picolinimidamido)-3-chlorophenyl)carbamoyl)-2-chlorophenyl)amino)(pyridin-2-yl)methylene)carbamate (2d)**. The reaction was carried out with **5d** (79 mg, 0.3 mmol), 7 (273 mg, 1.1 mmol), HgCl₂ (293 mg, 1.1 mmol), Et₃N (0.2 mL, 1.1 mmol) and anhydrous dichloromethane (2 mL) following the general method. The product was purified by reverse phase column chromatography (C18) with a 12 g cartridge using H₂O:CH₃NC (100:0 → 0:100), obtaining a yellowish solid (61 mg, 32 %). ¹H NMR (500 MHz, DMSO-*d*₆) δ 10.28 (s, 1H), 9.38 (s, 1H), 8.86 - 8.57 (m, 3H), 8.55 - 8.39 (m, 1H), 8.19 - 7.79 (m, 4H), 7.76 - 7.46 (m, 4H), 7.37 - 7.21 (m, 1H), 7.18 - 6.92 (m, 2H), 1.22 (br s, 18H). ¹³C NMR (126 MHz, DMSO-*d*₆) δ 163.6, 151.1, 148.5, 148.3, 141.6, 137.1, 128.6, 127.0, 125.5, 124.9, 123.1, 121.2, 120.7, 119.2, 80.4, 80.1, 27.6, 27.5. HPLC (UV) > 95 %. LRMS (ESI⁺): *m*/*z* 704.32 [M+H].

***tert*-butyl ((E)-((4-((4-((E)-AT-(tert-butoxycarbonyl)picolinimidamido)-2-chlorophenyl)carbamoyl)-3-chlorophenyl)amino)(pyridin-2-yl)methylene)carbamate (2e).** The reaction was carried out with **5e** (112 mg, 0.38 mmol), **7** (240 mg, 0.95 mmol), HgCl₂ (248 mg, 0.91 mmol), Et₃N (154 mg, 1.5 mmol) and anhydrous dichloromethane (3 mL) following the general method. The product was purified by circular chromatography with a silica plate (2 mm) previously neutralised with *n*-hexane (235 mL) and Et₃N (15 mL); eluent: hexane:EtOAC (6:4 --+ 4:6). The product was obtained as a yellowish solid (181 mg, 67 %). ¹H NMR (500 MHz, DMSO-*d*₆) δ 10.17 (s, 1H), 9.26 (s, 1H), 8.85 - 8.54 (m, 2H), 8.25 - 7.97 (m, 3H), 7.72 (ddd, *J* = 6.8, 4.8, 1.9 Hz, 2H), 7.33 (d, *J* = 8.3 Hz, 1H), 7.17 (d, *J* = 8.8 Hz, 1H), 6.66 (d, *J* = 2.4 Hz, 1H), 6.62 (d, *J=* 1.7 Hz, 1H), 6.52 (m, 2H), 5.76 (s, 1H), 5.34 (s, 1H), 1.48 (s, 18H). ¹³C NMR (126 MHz, DMSO-*d*₆) δ 162.0, 151.4, 149.8, 149.0, 148.6, 148.1, 148.0, 138.5, 131.5, 130.8, 129.6, 128.7, 128.0, 127.4, 122.9, 122.6, 122.2, 113.7, 113.4, 112.6, 111.6, 81.5, 81.0, 28.2, 27.6.

***tert*-butyl ((*E*)-((4-((4-((*E*)-*N*'-(tert-butoxycarbonyl)picolinimidamido)-3-chlorophenyl)carbamoyl)-3-chlorophenyl)amino)(pyridin-2-yl)methylene)carbamate (2f).** The reaction was carried out with **5f** (109 mg, 0.37 mmol), **7** (235 mg, 0.93 mmol), HgCl₂ (242 mg, 0.89 mmol), Et₃N (150 mg, 1.5 mmol) and anhydrous dichloromethane (2 mL) following the general method. The product was purified by circular chromatography with a silica plate (2 mm) previously neutralised with n-hexane (235 mL) and Et₃N (15 mL); eluent: CH₂Cl₂:EtOAC (95:5 -> 90:10). The product was obtained as a yellowish solid (32 mg, 12 %). ¹H NMR (300 MHz, DMSO-*d*₆) δ 10.53 (s, 1H), 10.10 (br s, 2H), 9.58 (m, 1H), 9.37 (m, 1H), 8.75 - 8.66 (m, 2H), 8.67 - 8.57 (m, 1H), 8.15 - 7.93 (m, 3H), 7.85 - 7.76 (m, 1H), 7.73 - 7.66 (m, 1H), 7.65 - 7.52 (m, 3H), 7.06 - 6.92 (m, 1H), 1.24 (s, 9H), 1.22 (s, 9H). ¹³C NMR (101 MHz, DMSO-*d*₆) δ 165.7, 160.8, 160.0, 156.3, 153.2, 151.1, 144.8, 141.7, 138.5, 137.3, 133.6, 129.5, 125.7, 123.1, 120.6, 119.9, 118.8, 113.6, 113.1, 80.1, 79.2, 27.5.

***tert*-butyl ((*E*)-((4-((4-((*E*)-*N'*-(*tert*-butoxycarbonyl)picolinimidamido)-2-isopropoxyphenyl)carbamoyl)-3-isopropoxyphenyl)amino)(pyridin-2-yl)methylene)carbamate (2g)**. The reaction was carried out with **5g** (50 mg, 0.15 mmol), **7** (96 mg, 0.38 mmol), HgCl₂ (98 mg, 0.36 mmol), Et₃N (61 mg, 0.6 mmol) and anhydrous dichloromethane (3 mL) following the general method. The reaction mixture was filtered over florisil, washing the precipitate with CH₂Cl₂ and MeOH. The organic phase was washed successively with H₂O and brine, dried on Na₂SO₄ and the solvent was removed under vacuum. The product was purified by circular chromatography with a silica plate (2 mm) previously neutralised with *n*-hexane (235 mL) and Et₃N (15 mL); eluent: hexane:EtOAC (2:3). The product was obtained as a yellowish solid (56 mg, 50 %). ¹H NMR (500 MHz, DMSO-*d*₆) δ 10.06 (s, 1H), 9.91 (s, 1H), 9.80 (s, 1H), 8.70 (dd, *J* = 9.9, 4.8 Hz, 2H), 8.33 (d, *J* = 8.8 Hz, 1H), 8.10 - 7.95 (m, 3H), 7.77 - 7.65 (m, 3H), 7.59 (ddd, *J* = 12.4, 7.9, 5.1 Hz, 3H), 7.48 (s, 1H), 7.37 (d, *J* = 7.5 Hz, 1H), 4.72 (hept, *J* = 6.0 Hz, 1H), 4.59 (d, *J* = 6.0 Hz, 1H), 1.47 (d, *J* = 6.0 Hz, 6H), 1.39 (d, *J* = 6.0 Hz, 6H), 1.25 (s, 9H), 1.22 (s, 9H).

***tert*-butyl ((*E*)-((4-((4-((*E*)-*N'*-(*tert*-butoxycarbonyl)picolinimidamido)-2-fluorophenyl)carbamoyl)-2-fluorophenyl)amino)(pyridin-2-yl)methylene)carbamate (2i).** The reaction was carried out with **5i** (51 mg, 0.19 mmol), **7** (195 mg, 0.77 mmol), HgCl₂ (211 mg, 0.78 mmol), Et₃N (107 µL, 0.77 mmol) and anhydrous dichloromethane (2 mL) following the general method. The reaction mixture was filtered over Celite and florisil, washing the precipitate with CH₂Cl₂ and MeOH. The solvent was removed under vacuum and the product was purified by reverse phase chromatography (12g, C-18). The reagents and secondary products elute with H₂O/CH₃CN: 90/10→40/60 while the desired product 2i elutes with 100% DMSO. The product was obtained as a yellowish solid (23 mg, 18 %). ¹H NMR (500 MHz, DMSO-*d*₆) δ 10.09 - 9.92 (m, 2H), 8.70 (d, *J* = 4.8 Hz, 1H), 8.59 - 8.47 (m, 1H), 8.31 (s, 1H), 8.14 - 8.06 (m, 1H), 8.00 (dd, *J* = 7.8, 1.8 Hz, 1H), 7.92 - 7.66 (m, 5H), 7.63 - 7.47 (m, 4H), 7.24-7.05 (m, 1H), 1.24 (s, 18H). ¹³C NMR (126 MHz, DMSO-*d*₆) δ 160.6, 156.8, 154.9, 152.2, 151.9, 149.6, 138.6, 137.6, 127.9, 127.8, 126.1, 126.0, 123.6, 123.5, 116.6, 115.6, 108.4, 108.2, 106.5, 79.6, 79.4, 28.0, 27.9. LRMS (ESI⁺) *m*/*z* 672 [M+H]⁺.

***tert*-butyl ((*E*)-((4-((4-((*E*)-*N'*-(*tert*-butoxycarbonyl)picolinimidamido)-3-fluorophenyl)carbamoyl)-2-fluorophenyl)amino)(pyridin-2-yl)methylene)carbamate (2j).** The reaction was carried out with **5j** (51 mg, 0.19 mmol), **7** (197 mg, 0.78 mmol), HgCl₂ (217 mg, 0.80 mmol), Et₃N (109 µL, 0.77 mmol) and anhydrous dichloromethane (2 mL) following the general method. The reaction mixture was filtered over Celite and florisil, washing the precipitate with CH₂Cl₂ and MeOH. The solvent was removed under vacuum and the product was purified by reverse phase chromatography (12g, C-18). The reagents and secondary products elute with H₂O/CH₃CN: 90/10→40/60 while the desired product **2i** elutes with 100% DMSO. The product was obtained as a yellowish solid (28.2 mg, 22 %). ¹H NMR (300 MHz, DMSO-*d*₆) δ 10.09 - 9.92 (m, 3H), 8.82 - 8.44 (m, 2H), 8.14 - 6.95 (m, 12H), 1.22 (s, 18H). ¹³C NMR (126 MHz, DMSO-*d*₆) δ 164.4, 164.2, 162.5, 153.5, 152.0, 151.9, 151.6, 151.3, 149.4, 149.3, 149.1, 149.0, 148.8, 138.9, 137.6, 128.5, 126.0, 124.5, 123.7, 123.4, 123.1, 116.4, 116.2, 116.2, 108.4, 108.2, 108.0, 80.9, 80.6, 28.0, 27.9. HPLC-MS (UV): > 95 %. LRMS (ESI⁺) *m*/*z* 672 [M+H]⁺.

***tert*-butyl ((*E*)-((4-((4-((*E*)-*N'*-(*tert*-butoxycarbonyl)picolinimidamido)-2-fluorophenyl)carbamoyl)-3-fluorophenyl)amino)(pyridin-2-yl)methylene)carbamate (2k)**. The reaction was carried out with **5k** (50 mg, 0.19 mmol), **7** (194 mg, 0.77 mmol), HgCl₂ (208 mg, 0.77 mmol), Et₃N (110 µL, 0.79 mmol) and anhydrous dichloromethane (2 mL) following the general method. The reaction mixture was filtered over Celite and florisil, washing the precipitate with CH₂Cl₂ and MeOH. The solvent was removed under vacuum and the product was purified by reverse phase chromatography (12g, C-18). The reagents and secondary products elute with H₂O/CH₃CN: 90/10→40/60 while the desired product **2k** elutes with 100% DMSO. The product was obtained as a yellowish solid (48.4 mg, 38 %). ¹H NMR (500 MHz, DMSO-*d*₆) δ 10.19 (s, 1H), 9.99 (s, 1H), 9.88 (d, *J* = 3.3 Hz, 1H), 8.70 (tt, *J* = 5.5, 1.1 Hz, 2H), 8.21 - 7.96 (m, 3H), 7.83 (t, *J* = 13.6 Hz, 2H), 7.76 (t, *J* = 8.7 Hz, 1H), 7.73 - 7.66 (m, 2H), 7.63 (d, *J* = 8.3 Hz, 1H), 7.61 - 7.53 (m, 2H), 7.52 (d, *J* = 8.3 Hz, 1H), 1.25 - 1.22 (m, 18H). ¹³C NMR (126 MHz, DMSO-*d*₆) δ 162.6, 160.6, 160.4, 156.8, 156.7, 151.9, 151.5, 149.6, 149.5, 149.1, 149.0, 148.9, 137.7, 137.6, 131.3, 126.2, 126.0, 123.6, 123.5, 121.4, 121.3, 116.6, 116.4, 108.3, 108.1, 107.7, 107.5, 79.8, 79.4, 28.01, 27.99. HPLC (UV): > 95 %. LRMS (ESI⁺): *m*/*z* 672 [M+H].

***tert*-butyl ((E)-((4-((4-((E)-Mo'-(tert-butoxycarbonyl)picolinimidamido)-3-fluorophenyl)carbamoyl)-3-fluorophenyl)amino)(pyridin-2-yl)methylene)carbamate (2l)**. The reaction was carried out with **5l** (50 mg, 0.19 mmol), **7** (194 mg, 0.77 mmol), HgCl₂ (214 mg, 0.79 mmol), Et₃N (106 µL, 0.76 mmol) and anhydrous dichloromethane (2 mL) following the general method. The reaction mixture was filtered over Celite and florisil, washing the precipitate with CH₂Cl₂ and MeOH. The solvent was stirred under vacuum and the product was purified by reverse phase chromatography (C-18). The reagents and secondary products elute with H₂O/CH₃CN: 90/10→40/60 while the desired product **2k** elutes with 100% DMSO. The product was obtained as a yellowish powder (27.8 mg, 22 %). ¹H NMR (500 MHz, DMSO-*d*₆) δ 10.56 - 10.33 (m, 1H), 10.31 -10.12 (m, 1H), 9.61 - 9.39 (m, 1H), 8.72 - 8.63 (m, 2H), 8.13 - 7.36 (m, 10H), 7.35 - 6.45 (m, 1H), 1.26 (s, 9H), 1.22 (d, *J* = 5.7 Hz, 9H). ¹³C NMR (126 MHz, DMSO-*d*₆) δ 162.7, 160.5, 160.4, 158.5, 156.7, 153.5, 152.0, 151.6, 151.52, 151.46, 151.1, 149.6, 149.3, 148.7, 137.7, 137.6, 130.9, 126.2, 125.9, 123.6, 123.4, 123.2, 116.4, 115.9, 115.8, 107.8, 107.6, 80.6, 79.8, 28.03, 27.99, 27.92. HPLC (UV): > 95 %. LRMS (ESI⁺): *m*/*z* 672 [M+H].

### 1.9. General method for the hydrolysis of the Boc group using trifluoroacetic acid (3b-g; 3i-l)

Trifluoroacetic acid (2 mL) is slowly added to a stirred solution in CH₂Cl₂ (2 mL) of the Boc-protected compound cooled with an ice bath. The resulting mixture is stirred for 2 hours at 0 °C. Excess trifluoroacetic acid and dichloromethane are evaporated under vacuum. The process is repeated, adding CH₂Cl₂ to the crude and evaporating the solvent under high vacuum. The sticky solid is ground with diethyl ether to precipitate the product as a powdered solid.

**5-(picolinimidamido)-*N*-(5-(picolinimidamido)pyridin-2-yl)picolinamide di-trifluoroacetate (3b)**. Following the general method with **5b** (80 mg; 0.13 mmol) **3b** was obtained as a brown solid (78 mg; 90 %). ¹H NMR (500 MHz, DMSO-*d*₆) δ 11.68 (br s, 1H), 10.55 (s, 1H), 9.44 (br s, 1H), 8.95 - 8.87 (m, 2H), 8.85 - 8.79 (m, 2H), 8.48 - 8.40 (m, 5H)), 8.27 (d, *J* = 8.1 Hz, 2H), 8.22 (d, *J* = 9.9 Hz, 1H), 7.98 - 7.80 (m, 4H). HPLC (UV): > 95 %. LRMS (ESI⁺): *m*/*z* 438.3 [M+H].

**3-chloro-*N*-(2-chloro-4-(picolinimidamido)phenyl)-4-(picolinimidamido)benzamide di-trifluoroacetate (3c).** Following the general method with **5c** (50 mg; 70 µmol) **3c** was obtained as a yellowish solid (45 mg, 88 %). ¹H NMR (500 MHz, DMSO-*d*₆) δ 10.41 (s, 1H), 10.09 (br s, 1H), 9.56 (br s, 1H), 8.91 (d, *J* = 4.4 Hz, 1H), 8.84 (d, *J* = 5.3 Hz, 1H), 8.42 - 8.32 (m, 2H), 8.32 - 8.20 (m, 2H), 8.17 (t, *J* = 7.8, 7.8 Hz, 1H), 8.09 (dd, *J* = 8.3, 2.0 Hz, 1H), 7.87 (dd, *J* = 8.3, 4.7 Hz, 1H), 7.82 - 7.73 (m, 3H), 7.61 - 7.46 (m, 2H), 3.63 (br s, 2H). ¹³C NMR (126 MHz, DMSO-*d*₆) δ 163.8, 159.6, 149.9, 149.4, 144.6, 138.5, 138.2, 134.9, 130.1, 129.6, 129.3, 128.7, 128.1, 127.2, 125.2, 123.9, 123.1. HPLC (UV) > 95 %. LRMS (ESI⁺): *m*/*z* 504.3 [M+H].

**3-chloro-*N*-(3-chloro-4-(picolinimidamido)phenyl)-4-(picolinimidamido)benzamide di-trifluoroacetate (3d)**. Following the general method with **5d** (25 mg; 36 µmol) **3d** was obtained as a yellowish solid (20 mg, 76 %). ¹H NMR (500 MHz, DMSO-*d*₆) δ 10.49 (br s, 3H), 9.14 - 8.78 (m, 3H), 8.61 - 8.28 (m, 3H), 8.25 - 8.04 (m, 3H), 8.03 - 7.68 (m, 3H), 7.36 - 7.17 (m, 3H). HPLC (UV) > 95 %. LRMS (ESI⁺): *m*/*z* 504.3 [M+H].

**2-chloro-*N*-(2-chloro-4-(picolinimidamido)phenyl)-4-(picolinimidamido)benzamide di-trifluoroacetate (3e)**. Following the general method with **5e** (57 mg; 80 µmol) **3e** was obtained as a brown solid (31 mg, 67 %). ¹H NMR (300 MHz, DMSO-*d*₆) δ 10.16 (br s, 1H), 9.94 (s, 1H), 9.85 (s, 2H), 9.49 (br s, 1H), 7.72 (d, *J* = 8.6 Hz, 2H), 7.53 - 7.40 (m, 5H), 7.33 - 7.24 (m, 2H), 7.13 (s, 1H), 6.85 (d, *J* = 2.1 Hz, 2H), 6.76 - 6.72 (m, 2H).

**2-chloro-*N*-(3-chloro-4-(picolinimidamido)phenyl)-4-(picolinimidamido)benzamide di-trifluoroacetate (3f)**. Following the general method with 5f (30 mg; 40 µmol) **3f** was obtained as a whitish solid (24 mg, 82 %). ¹H NMR (400 MHz, DMSO-*d*₆) δ 11.01 (s, 1H), 8.92 - 8.90 (m, 1H), 8.89 - 8.87 (m, 1H), 8.46 - 8.31 (m, 2H), 8.29 - 8.15 (m, 3H), 7.90 - 7.81 (m, 2H), 7.81 - 7.75 (m, 2H), 7.69 (br s, 1H), 7.58 (d, *J* = 8.6 Hz, 1H), 7.50 (br s, 1H), 3.52 (brs, 4H). ¹³C NMR (101 MHz, DMSO-*d*₆) δ 164.9, 159.8, 150.0, 149.9, 149.7, 149.5, 144.3, 140.3, 138.5, 138.3, 131.0, 130.9, 130.2, 129.6, 128.7, 128.3, 124.5, 123.6, 120.6, 119.5. LRMS (ESI⁺): *m*/*z* 405.4 [M+H].

**2-isopropoxy-*N*-(2-isopropoxy-4-(picolinimidamido)phenyl)-4-(picolinimidamido)benzamide di-hydrochloride (3g)**. A solution of HCl (4M in dioxane) was added dropwise to a solution of the compound **5g** in CH₂CL₂ (3 mL) cooled with an ice bath. The resulting mixture was stirred for 2 hours at 0°C. Excess HCl and dichloromethane are evaporated under vacuum. The sticky solid is ground with Et₂Or to precipitate the product as a brown solid (24 mg, 65 %). ¹H NMR (400 MHz, DMSO-*d*₆) δ 9.83 (s, 1H), 9.21 (s, 1H), 8.89 (d, *J* = 4.8 Hz, 1H), 8.74 - 8.61 (m, 1H), 8.40 (dd, J = 8.5, 4.6 Hz, 1H), 8.22 (ddd, *J =* 11.2, 6.4, 2.5 Hz, 1H), 8.17 - 7.97 (m, 1H), 7.85 (dd, *J* = 7.7, 4.8 Hz, 1H), 7.77 (dd, *J* = 9.9, 8.5 Hz, 2H), 7.14 (d, *J* = 2.3 Hz, 1H), 6.93 (dd, *J* = 8.5, 2.3 Hz, 1H), 6.45 - 6.41 (m, 1H), 6.36 - 6.32 (m, 1H), 4.75 - 4.58 (m, 4H), 3.73 - 3.64 (m, 1H), 3.52 - 3.43 (m, 1H), 1.44 - 1.36 (m, 12H). ¹³C NMR (101 MHz, DMSO-*d*₆) δ 163.2, 159.5, 157.3, 149.8, 149.0, 148.6, 148.3, 147.4, 147.1, 144.5, 138.5, 138.4, 137.9, 133.1, 129.1, 128.6, 128.0, 126.5, 123.8, 122.6, 122.0, 121.4, 114.9, 108.6, 107.8, 72.2, 71.9, 22.0, 21.9.

**3-fluoro-*N*-(2-fluoro-4-(picolinimidamido)phenyl)-4-(picolinimidamido)benzamide di-trifluoroacetate (3i).** Following the general method with **5i** (6.5 mg; 0.01 mmol) **3i** was obtained as a yellowish solid (5.5 mg, 97 %). ¹H NMR (300 MHz, DMSO-*d*₆) δ 10.24 (s, 1H), 9.44 (br s, 1H), 8.89 (d, *J* = 4.0 Hz, 1H), 8.76 (d, *J* = 3.5 Hz, 1H), 8.37 (d, *J* = 7.9 Hz, 3H), 8.23 (t, *J=* 7.8 Hz, 1H), 8.11 (t, J = 7.7 Hz, 1H), 8.02 - 7.67 (m, 7H), 7.46 (d, *J* = 10.8 Hz, 1H), 7.40 - 7.24 (m, 2H). HPLC-MS (UV) > 95 %; LRMS (ESI⁺) *m*/*z* 472 [M+H].

**3-fluoro-*N*-(3-fluoro-4-(picolinimidamido)phenyl)-4-(picolinimidamido)benzamide di-trifluoroacetate (3j).** Following the general method with **5j** (10.7 mg; 0.016 mmol) **3j** was obtained as a yellowish solid (7.5 mg, 80 %). ¹H NMR (300 MHz, DMSO-*d*₆) δ 10.24 (s, 1H), 9.44 (br s, 1H), 8.89 (d, *J* = 4.0 Hz, 1H), 8.76 (d, *J* = 3.5 Hz, 1H), 8.37 (d, *J* = 7.9 Hz, 3H), 8.23 (t, *J* = 7.8 Hz, 1H), 8.11 (t, *J* = 7.7 Hz, 1H), 8.02 - 7.67 (m, 7H), 7.46 (d, *J* = 10.8 Hz, 1H), 7.40 - 7.24 (m, 2H), ¹³C NMR (126 MHz, DMSO-*d*₆) δ 165.2, 158.8, 157.9, 155.9, 151.0, 150.2, 146.1, 139.9, 139.5, 129.8, 129.0, 128.3, 126.1, 125.0, 124.0, 123.0, 117.4, 117.2, 115.1. HPLC (UV); > 95 %. LRMS (ESI⁺) *m*/*z* 472 [M+H]⁺.

**2-fluoro-*N*-(2-fluoro-4-(picolinimidamido)phenyl)-4-(picolinimidamido)benzamide di-trifluoroacetate (3k).** Following the general method with **5k** (15.3 mg; 0.023 mmol) **3k** was obtained as a yellowish solid (12.6 mg, 94 %). ¹H NMR (300 MHz, DMSO-*d*₆) δ 10.25 (br s, 1H), 8.83 (s, 2H), 8.47 (s, 1H), 8.30 (s, 4H), 7.89 (s, 5H), 7.13 (s, 6H). ¹³C NMR (126 MHz, DMSO-*d*₆) δ 164.9, 163.6, 161.6, 160.9, 160.7, 160.5, 160.2, 152.3, 152.1, 134.4, 131.4, 131.0, 128.6, 126.6, 123.5, 116.5, 105.4. HPLC-MS (UV) > 95 %; LRMS (ESI⁺) *m*/*z* 472 [M+H].

**2-fluoro-N-(3-fluoro-4-(picolinimidamido)phenyl)-4-(picolinimidamido)benzamide di-trifluoroacetate (3l)**. Following the general method with **5l** (12.7 mg; 0.019 mmol) **3l** was obtained as a yellowish solid (10.3 mg, 93 %). ¹H NMR (300 MHz, DMSO-*d*₆) δ 11.72 (br s, 1H), 10.90 (s, 1H), 10.06 (br s, 1H), 9.30 (br s, 2H), 8.89 (t, *J* = 5.8 Hz, 2H) , 8.38 (d, *J* = 8.1 Hz, 2H), 8.30 - 8.17 (m, 2H), 7.97 (d, *J* = 12.8 Hz, 1H), 7.86 (dt, *J =* 13.9, 7.7 Hz, 3H), 7.65 (d, *J* = 8.9 Hz, 1H), 7.53 (t, *J* = 8.7 Hz, 1H), 7.45 (d, *J* = 9.8 Hz, 1H), 7.34 (d, *J* = 8.5 Hz, 1H). ¹³C NMR (126 MHz, DMSO-*d*₆) δ 165.4, 163.1, 161.1, 152.6, 152.2, 146.8, 141.3, 133.9, 133.8, 131.6, 131.4, 131.0, 126.4, 126.3, 124.0, 119.0, 119.0, 110.6, 110.4. HPLC-MS (UV) = 95 %; LRMS (ESI⁺) *m*/*z* 472 [M+H].

### 1.10. Method for the synthesis of 2-arylimidamides 3a, 3h, 17, 18

**4-(picolinimidamido)-N-(4-(picolinimidamido)phenyl)benzamide di-trifluoroacetate (3a).** A solution of the commercial diamine **5a** (0.5 g; 2.2 mmol) in EtOH/CH₃CN (3:1, 6 mL) was stirred at 0 °C. Next, a solution of **8** (2 g, 5.5 mmol) in EtOH/CH₃CN (3:1, 6 mL) was slowly added. The reaction mixture was stirred at room temperature for 48 h. The solvents were evaporated under vacuum and the product was purified by silica column chromatography eluting with hexane:EtOAc (20:80 → 0:100), obtaining a yellowish solid (220 mg; 23 %). ¹H NMR (400 MHz, DMSO-*d*₆) δ 10.05 (s, 1H), 8.65 (ddt, *J* = 6.7, 4.9, 1.4, 1.4 Hz, 2H), 8.33 (d, *J* = 8.0 Hz, 2H), 8.07 - 7.88 (m, 4H), 7.79 (d, *J* = 8.7 Hz, 2H), 7.56 (dddd, *J* = 9.0, 7.5, 4.8, 1.3 Hz, 2H), 7.05 (d, *J* = 8.0 Hz, 2H), 6.95 (d, *J* = 8.2 Hz, 2H), 6.90 - 6.00 (m, 4H). ¹³C NMR (101 MHz, DMSO-*d*₆) δ 164.8, 153.7, 151.8, 151.6, 151.2, 148.1, 148.0, 145.6, 137.2, 137.1, 134.2, 129.0, 128.6, 125.6, 125.4, 121.6, 121.5, 121.4, 121.2, 113.7, 112.5. HPLC (UV): > 95%. LRMS (ESI⁺): *m*/*z* 436.4 [M+H].

**4-(picolinimidamido)-*N*-(5-(picolinimidamido)pyridin-2-yl)benzamide di-hydrochloride (3h).** A solution of **5h** (86 mg; 0.38 mmol) in EtOH/CH₃CN (3:1, 8 mL) was stirred at 0 °C. Next, the solid 8 (284 mg, 0.79 mmol) was added in one go. The reaction mixture was stirred at 0 °C and then allowed to return to room temperature, continuing stirring for 5 days. Et₂O was added to the reaction mixture and the precipitate was collected on a filter plate. The precipitate was washed with EtzO, and then dissolved in EtOH (20 mL). The solution was cooled with an ice bath and basified (pH ≈ 10) with NaOH 1N. Next, the ethanol was evaporated under vacuum and water (25 mL) was added to the crude. The aqueous phase was extracted with EtOAc (3×40 mL). The organic phase was washed with brine and dried over Na₂SO₄. The solvent was evaporated, obtaining a yellowish residue. A first purification by silica chromatography (5 g SI cartridge) with CHCl₃/ NH_{3(sat.)}-MeOH: 0->5 % yielded a mixture (≈75/25, 38 mg) of the expected product 3h (M = 436) together with the mono-substituted product (M = 332). The mixture was dissolved in CH₂Cl₂/MeOH (2 mL), cooled with an ice bath, and treated with a saturated HCl_{g} solution in dioxane. The reaction was stirred gently at 0 °C for 1 h. The precipitate (i.e., hydrochloride of the mono-substituted product, white solid, 20 mg) was collected by filtration. The filtrate was evaporated and the solid obtained was recrystallised with CH₂Cl₂/MeOH at -20 °C. The product was washed with Et₂O, obtaining a whitish solid (10 mg). HPLC = 91 %. ¹H NMR (400 MHz, DMSO-*d*₆) δ 11.99 (brs, 1H), 11.84 (brs, 1H), 11.27 (s, 1H), 10.22 (brs, 1H), 10.20 (brs, 1H), 9.50 (brs, 2H), 8.92 (d, *J* = 4.7 Hz, 2H), 8.55 (d, *J* = 2.7 Hz, 1H), 8.50 (d, *J* = 8.0 Hz, 1H), 8.47 (d, *J* = 9.2 Hz, 1H), 8.41 (d, *J* = 8.8 Hz, 1H), 8.31-8.20 (m, 4H), 8.01 (dd, *J* = 8.8, 2.7 Hz, 1H), 7.92 - 7.84 (m, 2H), 7.65 (d, *J* = 8.1 Hz, 2H). LRMS (ESI⁺) *m*/*z* 437 [M+H]⁺.

***N,N"*-(ethane-1,2-diylbis(4,1-phenylene))dipicolinimidamide (17).** A suspension of 4,4'-diaminobibenzyl **16** (106 mg, 0.5 mmol) and naftalen-2-ylmethyl pyridine-2-carbimidotioate **8** (449 mg, 1.25 mmol) in EtOH/CH₃CN (3:1, 16 mL) was stirred overnight. The solvent was evaporated under vacuum and the product was purified by silica column chromatography eluting with hexane:EtOAc (100:0 → 10:90). The product **17** was obtained as a yellowish solid (51 mg, 24 %). ¹H NMR (500 MHz, DMSO-*d*₆) δ 8.63 (ddd, *J* = 4.8, 1.8, 1.1 Hz, 2H), 8.31 (d, *J* = 7.8 Hz, 2H), 7.95 (td, *J* = 7.8, 7.8, 1.8 Hz, 2H), 7.55 (ddd, *J* = 7.8, 4.8, 1.1 Hz, 2H), 7.33 - 7.17 (m, 4H), 6.88 (d, *J* = 7.8 Hz, 4H), 2.87 (s, 4H). ¹³C NMR (126 MHz, DMSO-*d*₆) δ 151.8, 151.4, 148.0, 147.5, 137.1, 135.6, 129.2, 125.4, 121.4, 121.3, 36.9. HRMS: 95 %. Anal. Calc.: C, 74.26; H, 5.75; N, 19.99. It being found that: C, 73.59; H, 5.74; N, 19.36.

***N,N-*((carbonylbis(azanediyl))bis(4,1-phenylene))dipicolinimidamide (18).** A suspension of 1,3-bis(4-aminophenyl)urea **10** (300 mg, 1,2 mmol) and naphthalen-2-ylmethyl pyridine-2-carbimidothioate hydrobromide **8** (1.1 g, 3.1 mmol) in EtOH/CH₃CN (3:1, 24 mL) was stirred overnight. The solid obtained was washed with MeOH and dried. Next, the solid was dissolved in CH₂CL₂ and MeOH was added to precipitate the product as a whitish solid (220 mg, 39.4 %). ¹H NMR (400 MHz, DMSO-*d*₆) δ 7.90 - 7.88 (m, 4H), 7.88 - 7.86 (m, 4H), 7.86 - 7.84 (m, 2H), 7.83 - 7.81 (m, 4H), 7.80 - 7.77 (m, 2H), 7.66 - 7.64 (m, 2H), 7.53 - 7.48 (m, 4H). ¹³C NMR (101 MHz, DMSO) δ 150.9, 139.7, 133.0, 128.0, 127.5, 127.5, 127.4, 126.9, 126.2, 126.0, 125.7.

### Example 2. In vitro assays of the leishmanicidal activity of the compounds of the invention

The compounds were evaluated *in vitro* against promastigote forms of *L. donovani* (cepa MHOM/ET/67/HU3) following the MTT method described previously (Kennedy ML, et al. *J Med Chem* **2001,** *44*, 4668-4676) and intracellular amastigotes of the parasite *L*. *donovani* (strain MHOM/ET/67/HU3) infecting macrophages derived from THP-1 cells and applying the resazurin colorimetric method described previously (Koutsoni OS, et al. *Bio-protocol* **2019**;9:e3410). Furthermore, their cytotoxicity on human THP-1 cells was evaluated and the selectivity index (SI) of each compound was calculated (Table 1). All of these cell lines were cultured at 28 °C in RPMI 1640 modified medium (Invitrogen) supplemented with 10 % hiFBS (Invitrogen), as previously described (Jackson, PR, et al. Am. J. Trop. Med. Hyg. 1984, 33, 808- 819).

The compounds object of this patent show activities on promastigote and intracellular amastigote forms of *L. donovani* in the micromolar to submicromolar range **(3a, 3b, 3c, 3d, 3f, 3g, 3h, 17)**, being of similar potency to the reference drug miltefosine used in this trial. Of note are the compounds **3b, 3c, 3d** which are more effective on intracellular amastigote forms with respect to promastigote forms.

Furthermore, the compounds show selectivity towards *Leishmania* with selectivity indices (SI) > 19 against human THP-1 cells for the most active compounds **(3a, 3b, 3c, 3d, 17).** Compound **3a** stands out due to its submicromolar activity against promastigote and intracellular amastigote forms of *L. donovani,* as well as its selectivity index >76.

**Table 1.^{to} Leishmanicidal activity of the compounds of the invention (EC₅₀ represents the concentration of the compound to produce 50 % of the maximum effect of that compound)**

| Cmpd | EC₅₀ (µM) | | | Sl*^{b}* |
|---|---|---|---|---|
| | *L. donovani* promastigotes | *L. donovani* intracellular amastigotes | THP-1 cells | |
| **13** | 8.67 ± 3.84 | >10 | 14.10 ± 4.41 | 1.6 |
| **3a** | 0.26 ± 0.05 | 0.65 ± 0.20 | >50 | > 76.9 |
| **3b** | 0.97 ± 0.24 | 0.55 ± 0.06 | 11.15 ± 3.33 | 20.3 |
| **3c** | 1.63 ± 0.29 | 0.91 ± 0.30 | 18.59 ± 2.36 | 20.4 |
| **3d** | 1.44 ± 0.13 | 0.78 ± 0.11 | 16.42 ± 1.25 | 21.1 |
| **3f** | 0.89 ± 0.22 | 2.56 ± 0.41 | 49.44 ± 6.80 | 19.3 |
| **3g** | 3.00 ± 0.51 | 4.19 ± 0.57 | >50 | >11.9 |
| **3h** | 1.47 ± 0.46 | >10 | >50 | - |
| **17** | 0.33 ± 0.06 | 1.11 ± 0.23 | >50 | >45.0 |
| AmB*^{d}* | 0.07 ± 0.01 | 0.24 | 27.85 | 116.0 |
| MIL*^{e}* | 6.51 ± 0.35 | 0.91 ± 0.05 | 26.25 ± 2.58 | 28.8 |

| | | | | |
|---|---|---|---|---|
| ^{a} Parasites and THP-1 cells were cultured in the presence of increasing concentrations of the compounds. The data represent the mean ± standard deviation of 3 independent experiments. *^{b}* SI= selectivity index (EC₅₀ THP-1/EC₅₀ parasite (amastigote form)). *^{c}* not assayed. *^{d}*Amphotericin B. *^{e}*Miltefosine. | | | | |

### Example 3. In vitro assays of the trypanocidal activity of the compounds of the invention.

The compounds were evaluated *in vitro* against bloodtream trypomastigote forms of *T.b. brucei* (wild strain s427 and drug-resistant strain B48) following the Alamar blue colorimetric method described previously (Ebiloma GU, et al. Eur. J. Med. Chem. 2018, 150, 385-402). Moreover, the compounds were evaluated *in vitro* against epimastigotes and intracellular amastigotes of *T. cruzi* (CL strain lacZ) following a previously described protocol (Fonseca-Berzal C, et al. Eur. J. Med. Chem. 2016, 115, 295-310).

The compounds of the invention of the bis(2-aminoimidazolines) series show activities in the micromolar range against the sensitive strain (s427) and the drug-resistant strain B48 of the parasite *Trypanosoma brucei* (Table 2). Compounds of the bisarylimidamide series show activities in the submicromolar range against the susceptible strain (s427) and the drug-resistant strain B48. It should be noted that the compounds are equipotent against the sensitive and drug-resistant B48 strains (Resistance factor ≈1). The compounds **3a, 3b,** and **3d** in particular are the most potent of the series with submicromolar activity and a selectivity index > 118.

Regarding the activity on the parasite *Trypanosoma cruzi* (Table 3), the compounds of the bisarylimidamide series stand out with activities on epimastigotes in the nanomolar **(3c, 3d, 3f),** submicromolar **(3a, 3b, 3i, 3j, 3k, 3l)** and low micromolar **(3e, 3g, 17)** range. These compounds are also active on the intracellular amastigote form of *T. cruzi,* with activities in the submicromolar **(3c, 3d, 3F)** to low micromolar **(3a, 3g, 17)** range. It is worth highlighting the compound **3f,** which is twice as potent as the reference drug benznidazole, and the compound **3c,** which has the same potency as benznidazole. All compounds are selective towards the parasite with selectivity indices between 24 and 399 against intracellular amastigotes.

**Table 2.^{a} Activity on T. brucei (EC₅₀) of the compounds of the invention and cytotoxicity (CC₅₀: is the cytotoxic concentration: the concentration that causes 50 % cell death) on HEK cells.**

| Cmpd | EC₅₀ (µM) | | CC₅₀ (µM) | SI |
|---|---|---|---|---|
| | *T. brucei^{b}* | *T. brucei B48^{c}* | HEK | |
| **1a** | 85.6 ± 2.3 | 81.9 ± 8.5 (0.79)*^{d}* | >100 | |
| **1b** | 11.3 ± 0.2 | 11.6 ± 0.3 (1.03) | 80.9 ± 2.7 | 7.2 |
| **1c** | 1.96 ± 0.03 | 1.93 ± 0.04 (0.99) | 16.8 ± 0.9 | 8.6 |
| **1d** | 1.68 ± 0.06 | 1.81 ± 0.06 (1.08) | 20.3 ± 1.9 | 12 |
| **1e** | 5.56 ± 0.26 | 5.75 ± 0.11 (1.03) | 16.2 ± 0.5 | 2.9 |
| **1f** | 2.64 ± 0.09 | 2.93 ± 0.17 (1.11) | 10.2 ± 0.3 | 3.9 |
| **13** | 2.3 ± 0.2 | 2.2 ± 0.2 | 13.5 ± 0.1 | 5.9 |
| **14** | 57.1 ± 1.4 | 68.8 ± 1.4 (1.2) | >200 | >3.5 |
| **3a** | 0.708 ± 0.061 | 0.68 ± 0.06 (0.96) | >200 | >282 |
| **3b** | 0.12 ± 0.01 | 0.35 ± 0.02 (2.9) | 14.2 ± 0.8 | 118 |
| **3c** | 10.5 ± 1.9 | 12.5 ± 2.0 (1.19) | 46.0 ± 0.1 | 4.4 |
| **3d** | 0.25 ± 0.04 | 0.69 ± 0.16 (2.73) | 33.4 ± 3.1 | 133 |
| **3e** | 15.5 ± 2.7 | 28.1 ± 2.6 (1.81) | >100 | >6.5 |
| **3f** | 9.4 ± 0.96 | 7.6 ± 0.8 | 35.1 ± 3.5 | 3.7 |
| **3g** | 5.07 ± 0.03 | 3.96 ± 0.22 (0.78) | 69.0 ± 1.7 | 13.6 |
| **3h** | 23.7±0.7 | 24.010.4 | >100 | >4.2 |
| pent.*^{e}* | 0.0033 ± 0.0006 | 0.55 ± 0.03 (168) | | |
| dimin.*^{f}* | 0.101 ± 0.005 | 1.33 ± 0.14 (13.1) | | |

| | | | | |
|---|---|---|---|---|
| *^{a}* The data represent the mean ± standard deviation of 3 independent experiments. *^{b}* Strain s427 of *T. b. brucei. ^{c}* Strain B48 of *T. b. brucei* resistant to pentamidine and diminazene. *^{d}* RF = resistance factor (EC₅₀ *T. b.* B48 / EC₅₀ *T. b.* s427). *^{e}* Pentamidine. *^{f}* Diminazene. | | | | |

**Table 3.^{a} Activity on T. cruzi (EC₅₀, µM) of the compounds of the invention**

| Cmpd | *T. cruzi* epimastigotes | *T. cruzi* intracellular amastigotes | L929 cells (72 h) | SI epi*^{b}* | SI amast |
|---|---|---|---|---|---|
| **3a** | 0.21 ± 0.02 | 1.28 ± 0.34 | 89.1 ± 8.3 | 424 | 69.6 |
| **3b** | 0.35 ± 0.07 | nd | 0.79 ± 0.08 | 2.26 | |
| **3c** | 0.03 ± 0.00 | 0.58 ± 0.09 | 44.85 ± 7.59 | 1495 | 77.3 |
| **3d** | 0.06 ± 0.00 | 0.55 ± 0.17 | 9.55 ± 1.50 | 159 | 17.4 |
| **3e** | 32.65 ± 1.75 | nd | >200 | >6.1 | |
| **3f** | 0.03 ± 0.00 | 0.26 ± 0.04 | 103.6 ± 10.3 | 3454 | 399 |
| **3g** | 7.52 ± 0.26 | 3.51 ± 0.08 | 85.15 ± 4.76 | 11.3 | 24.3 |
| **3i** | 0.22 ± 0.02 | nd | 7.51 ± 1.65 | 34.1 | |
| **3j** | 0.53 ± 0.08 | nd | < 6.25 | < 11.8 | |
| **3k** | 0.47 ± 0.04 | nd | 15.91 ± 0.66 | 33.9 | |
| **3l** | 0.15 ± 0.01 | nd | 24.71 ± 4.05 | 165 | |
| **17** | 4.33 ± 0.65 | 7.66 ± 0.75 | *>*200 | >46 | >26.1 |
| benzn*^{c}* | 25.27 ± 2.10 | 0.54 ± 0.01 | >200 | >7.9 | >370 |

| | | | | | |
|---|---|---|---|---|---|
| *^{a}* Parasites (strain CL *lac*Z) and L929 cells were cultured in the presence of increasing concentrations of the compounds. The data represent the mean ± standard deviation of 3 independent experiments. *^{b}* Sl= selectivity index (EC₅₀ L929 / EC₅₀ parasite). *^{c}* Reference drug: benznidazole. | | | | | |

### Example 4. Stability of the compound JN-II-40 against hepatic and serum metabolism.

The microsomal stability of the compound **3a** towards metabolism by the cytochrome P450 (Phase I metabolism) and uridine glucuronosyl transferase (UGT) (Phase II metabolism) in the presence of NADPH (nicotinamide adenine dinucleotide phosphate) and UDPGA was studied (Table 4). To that end, the previously described protocol was followed (Manzano JI, et al. J.Med. Chem. 2019, 62, 10664-10675).

Compound **3a** was not metabolised by human liver and mouse liver microsomes (CD-1). Neither was it metabolised by enzymes using the cofactor UDPGA (Uridine 5'-diphosphoglucuronic acid) (phase 2 enzymes) from mouse liver and human liver microsomes. No significant metabolites in the reaction time of up to 120 min were found. Therefore, the compound **3a** is stable. Its half-life time was more than 2 h. In comparison, human liver microsomes rapidly metabolised (high intrinsic clearance) diclofenac under the same conditions with a half-life of less than 30 min. Comparatively, mouse liver microsomes (CD-1) slowly metabolised the drug diclofenac.

Compound **3a** was unchanged in human serum and was stable during 1 h of incubation. Then, it is concluded that, in the experimental conditions used, the compound is metabolically stable against microsomal fractions from humans and mice, and in human serum.

**Table 4. Hepatic and serum metabolism**

| Hepatic metabolism of the compound **3a** | |
|---|---|
| t_{1/2} mouse (microsomes, phase I/II) | > 2 h |
| | > 2 h |
| t_{1/2} human (microsomes, phase I) | > 2 h |
| t_{1/2} human (S9 fraction, phase II) | |
| Metabolism in human serum | > 1 h |

## Claims

1. A compound of general formula (I) 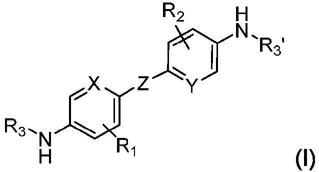
or any of the pharmaceutically acceptable isomers, solvates or salts thereof,
wherein:
R₁ and R₂ are independently selected from H, O'Pr and halogen;
R₃ and R₃' are independently selected from 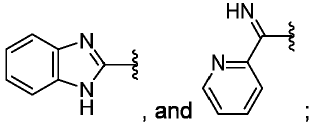
Z is selected from 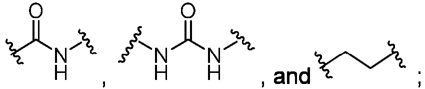
X and Y are independently selected from CH and N,
for use thereof as a medicament.

2. The compound for use, according to claim 1, wherein R₁ and R₂ are the same.

3. The compound for use, according to claim 2, wherein R₁ and R₂ are H.

4. The compound for use, according to claim 2, wherein R₁ and R₂ are Cl.

5. The compound for use, according to claim 2, wherein R₁ and R₂ are O'Pr.

6. The compound for use, according to claim 2, wherein R₁ and R₂ are F.

7. The compound for use, according to any of the preceding claims, wherein
X and Y are CH.

8. The compound for use, according to any of the preceding claims 1 to 6, wherein X is CH and Y is N.

9. The compound for use, according to any of the preceding claims 1 to 6, wherein X and Y are both N.

10. The compound for use, according to any of the preceding claims, wherein
Z is

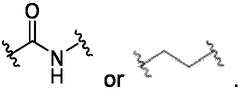

11. The compound for use, according to any of the preceding claims, wherein
R₃ and R₃' are the same and are :

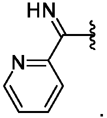

12. The compound for use, according to claim 1, wherein the compound is selected from:
- 4-(picolinimidamido)-*N*-(4-(picolinimidamido)phenyl)benzamide **(3a)**
- 5-(picolinimidamido)-*N-*(5-(picolinimidamido)pyridin-2-yl)picolinamide **(3b)**
- 3-chloro-*N*-(2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(2-chloro-4-(picolinimidamido)phenyl)-4-(picolinimidamido)benzamide **(3e)**
- 2-chloro-*N*-(3-chloro-4-(picolinimidamido)phenyl)-4-(picolinimidamido)benzamide **(3f)**
- 2-isopropoxy-*N*-(2-isopropoxy-4-(picolinimidamido)phenyl)-4-(picolinimidamido)benzamide **(3g)**
- 4-(picolinimidamido)-*N*-(5-(picolinimidamido)pyridin-2-yl)benzamide **(3h)**
- 3-fluoro-*N*-(2-fluoro-4-(picolinimidamido)phenyl)-4-(picolinimidamido)benzamide di-trifluoroacetate **(3i)**
- 3-fluoro*-N*-(3-fluoro-4-(picolinimidamido)phenyl)-4-(picolinimidamido)benzamide di-trifluoroacetate **(3j)**
- 2-fluoro-*N-*(2-fluoro-4-(picolinimidamido)phenyl)-4-(picolinimidamido)benzamide di-trifluoroacetate **(3k)**
- 2-fluoro-*N*-(3-fluoro-4-(picolinimidamido)phenyl)-4-(picolinimidamido)benzamide di-trifluoroacetate **(3l)**
- *N,N*"-(ethane-1,2-diylbis(4,1-phenylene))dipicolinimidamide **(17)**
- *N,N*"-((carbonylbis(azanediyl))bis(4,1-phenylene))dipicolinimidamide **(18)**
- 4-((1,3-dihydro-2*H*-benzo[*d*]imidazol-2-ylidene)amino)-*N*-(4-((1,3-dihydro-2*H-*benzo[*d*]imidazol-2-ylidene)amino)phenyl)benzamide **(1a)**
- 5-((1,3-dihydro-2*H*-benzo[d]imidazol-2-ylidene)amino)-*N*-(5-((1,3-dihydro-2*H-*benzo[*d*]imidazol-2-ylidene)amino)pyridin-2-il)picolinamide (1b)
- 3-chloro-*N*-(2-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1c)**
- 3-chloro-*N*-(3-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1d)**
- 2-chloro-*N*-(2-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1e)**
- 2-chloro-*N*-(3-chloro-4-((1,3-dihydro-2*H*-benzo[*d*]imidazol-2-ylidene)amino)phenyl)-4-((1,3-dihydro-2*H*-benzo[d]imidazol-2-ylidene)amino)benzamide **(1f)**
- -4,4'-(ethane-1,2-diyl)bis(*N*-(1*H*-benzo[*d*]imidazol-2(3*H*)-ylidene)aniline(**13**)
- 1,3-bis(4-((1,3-dihydro-2*H*-benzo[*d*]imidazol-2-ylidene)amino)phenyl)urea **(14)**.

13. A compound of general formula (I) described in any of claims 1 to 12, for use in the prevention and/or treatment of diseases caused by protozoan parasites.

14. The compound for use, according to claim 13, wherein the protozoan parasites are of the genera *Leishmania* or *Trypanosoma.*

15. The compound for use, according to claim 14, wherein protozoan parasites of the genera *Leishmania* or *Trypanosoma* are of the species *L. aethiopica, L. amazonensis, L. arabica, L. archibaldi, L. aristedesi, L. braziliensis, L. chagasi (syn. L. infantum), L. colombiensis, L. deanei, L. donovani, L. enriettii, L. equatorensis, L. forattinii, L. gamhami, L. gerbili, L. guyanensis, L. herreri, L. hertigi, L. infantum, L. killicki, L. lainsoni, L. major, L. mexicana, L. naiffi, L. panamensis, L. peruviana, L. pifanoi, L. shawi, L. tarentolae, L. tropica, L. turanica, L. venezuelensis, T. brucei, T. cruzi, T. congolense, T. equinum, T. equiperdum, T. evansi or T. vivax.*

16. The compound for use, according to claim 15, wherein the protozoan parasites are of the species *T. brucei, T. cruzi and*/*or L. donovani.*

17. The compound for use, according to any of the preceding claims 13 to 16, wherein the disease is selected from leishmaniasis, human African trypanosomiasis, animal tripanosomiasis or American tripanosomiasis.

18. A compound of general formula (I) 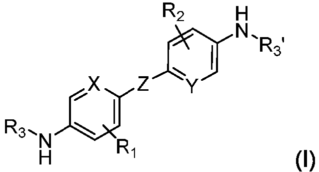
or any of the pharmaceutically acceptable isomers, solvates or salts thereof,
wherein:
R₁, R₂, R₃, R₃', Z, X and Y are described according to any of claims 1 to 10, and with the condition that the compound is not: 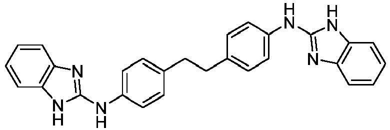 nor a tautomer thereof.

19. The compound according to claim 18, wherein the compound is selected from:
- 4-(picolinimidamido)-*N*-(4-(picolinimidamido)phenyl)benzamide **(3a)**
- 5-(picolinimidamido)-*N*-(5-(picolinimidamido)pyridin-2-yl)picolinamide **(3b)**
- 3-chloro-*N* (2-chloro-4-(picolinimidamido)phenyl)-4-(picolinimidamido)benzamide **(3c)**
- 3-chloro-*N*-(3-chloro-4-(picolinimidamido)phenyl)-4-(picolinimidamido)benzamide **(3d)**
- 2-chloro-*N*-(2-chloro-4-(picolinimidamido)phenyl)-4-(picolinimidamido)benzamide **(3e)**
- 2-chloro-*N*-(3-chloro-4-(picolinimidamido)phenyl)-4-(picolinimidamido)benzamide **(3f)**
- 2-isopropoxy-*N*-(2-isopropoxy-4-(picolinimidamido)phenyl)-4-(picolinimidamido)benzamide **(3g)**
- 4-(picolinimidamido)-*N*-(5-(picolinimidamido)pyridin-2-yl)benzamide **(3h)**
- 3-fluoro-*N*-(2-fluoro-4-(picolinimidamido)phenyl)-4-(picolinimidamido)benzamide di-trifluoroacetate **(3i)**
- 3-fluoro*-N*-(3-fluoro-4-(picolinimidamido)phenyl)-4-(picolinimidamido)benzamide di-trifluoroacetate **(3j)**
- 2-fluoro-*N-*(2-fluoro-4-(picolinimidamido)phenyl)-4-(picolinimidamido)benzamide di-trifluoroacetate **(3k)**
- 2-fluoro-*N*-(3-fluoro-4-(picolinimidamido)phenyl)-4-(picolinimidamido)benzamide di-trifluoroacetate **(3l)**
- *N*,*N*"-(ethane-1,2-diyI bis(4,1 -phenylene))dipicolinimidamide **(17)**
- *N,N*"-((carbonylbis(azanediyl))bis(4,1-phenylene))dipicolinimidamide **(18)**
- 4-((1,3-dihydro-2*H*-benzo[*d*]imidazol-2-ylidene)amino)-*N*-(4-((1,3-dihydro-2*H-*benzo[*d*]imidazol-2-ylidene)amino)phenyl)benzamide **(1a)**
- 5-((1,3-dihydro-2*H*-benzo[*d*]imidazol-2-ylidene)amino)-*N*-(5-((1,3-dihydro-2*H-*benzo[*d*]imidazol-2-ylidene)amino)pyridin-2-il)picolinamide (**1b**)
- 3-chloro-*N*-(2-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1c)**
- 3-chloro-*N*-(3-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1d)**
- 2-chloro-*N*-(2-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1e)**
- 2-chloro-*N*-(3-chloro-4-((1,3-dihydro-2*H*-benzo[*d*]imidazol-2-ylidene)amino)phenyl)-4-((1,3-dihydro-2*H*-benzo[*d*]imidazol-2-ylidene)amino)benzamide **(1f)**
- 1,3-bis(4-((1,3-dihydro-2*H*-benzo[*d*]imidazol-2-ylidene)amino)phenyl)urea **(14).**

20. A pharmaceutical composition comprising a compound of general formula (I) described according to any of claims 18 to 19, together with a pharmaceutically acceptable excipient, adjuvant and/or carrier.

21. The pharmaceutical composition, according to claim 20, which further comprises another antiparasitic agent.
